(19) Europäisches Patentamt
European Patent Office
Office européen des brevets

(11) **EP 1 957 524 B1**

(12) **EUROPEAN PATENT SPECIFICATION**

(45) Date of publication and mention
of the grant of the patent:
**17.02.2010 Bulletin 2010/07**

(21) Application number: **06821700.9**

(22) Date of filing: **16.11.2006**

(51) Int Cl.:
***C07K 14/435*** *(2006.01)*    ***A61K 38/17*** *(2006.01)*

(86) International application number:
**PCT/IN2006/000452**

(87) International publication number:
**WO 2007/057922 (24.05.2007 Gazette 2007/21)**

(54) **TARGETED FUSION PROTEINS FOR CANCER THERAPY**

ZIELGERICHTETE FUSIONSPROTEINE FÜR DIE KREBSTHERAPIE

PROTEINES HYBRIDES CIBLEES DESTINEES A LA CANCEROTHERAPIE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **18.11.2005 IN DE30842005**

(43) Date of publication of application:
**20.08.2008 Bulletin 2008/34**

(73) Proprietor: **Dabur Pharma Limited**
**Adjacent to Safdarjung Hospital**
**New Delhi 110 029 (IN)**

(72) Inventors:
• **MAITHAL, Kapil**
**Uttar Pradesh (IN)**
• **NACHIAPPAN, Dhatchana, Moorthy**
**Uttar Pradesh (IN)**
• **MUKHERJEE, Rama**
**Uttar Pradesh (IN)**

(74) Representative: **Dzieglewska, Hanna Eva**
**Dehns**
**St Bride's House**
**10 Salisbury Square**
**London**
**EC4Y 8JD (GB)**

(56) References cited:
**WO-A-98/49311**

• ANTIGNANI ANTONELLA ET AL: "A chimeric protein induces tumor cell apoptosis by delivering the human Bcl-2 family BH3-only protein bad" BIOCHEMISTRY, vol. 44, no. 10, March 2005 (2005-03), pages 4074-4082, XP002423212 ISSN: 0006-2960
• AZAR Y ET AL: "GnRH-Bik/Bax/Bak chimeric proteins target and kill adenocarcinoma cells" APOPTOSIS, LONDON, GB, vol. 5, no. 6, December 2000 (2000-12), pages 531-542, XP002303415 ISSN: 1360-8185
• LIU S ET AL: "Tumor cell-selective cytotoxicity of matrix metalloproteinase-activa ted ant hrax toxin" CANCER RESEARCH, AMERICAN ASSOCIATION FOR CANCER RESEARCH, BALTIMORE, MD, US, vol. 60, no. 21, 1 November 2000 (2000-11-01), pages 6061-6067, XP002311242 ISSN: 0008-5472
• FRANKEL A E ET AL: "Peptide toxins directed at the matrix dissolution systems of cancer cells" PROTEIN AND PEPTIDE LETTERS, BENTHAM SCIENCE PUBLISHERS, SCHIPHOL, NL, vol. 9, no. 1, February 2002 (2002-02), pages 1-14, XP002408612 ISSN: 0929-8665
• KREITMAN ROBERT J: "Immunotoxins for targeted cancer therapy." THE AAPS JOURNAL 2006, vol. 8, no. 3, 2006, pages E532-E551, XP002423213 ISSN: 1550-7416

SEQUENCE LISTING

<110> Dabur Pharma Limited
Maithal, Kapil
Nachiappan, Dhatchana Moorthy
Mukherjee, Rama

<120> TARGETED FUSION PROTEINS FOR CANCER THERAPY

<130> 3084-DEL-06

<160> 11

<170> PatentIn version 3.3

<210> 1
<211> 84
<212> DNA
<213> Artificial

<220>
<223> Artificially generated oligonucleotide (sense template) encoding the VIP peptide designed by optimizing degenerate codons for maximimal protein expression in E.coli

<400> 1

catagtgatg cagtgtttac cgataattat acgcgccttc gtaagcagat ggcggttaaa     60

aagtacttga atagcattct gaac     84

<210> 2
<211> 84
<212> DNA
<213> Artificial

<220>
<223> Artificially generated oligonucleotide (anti sense template) encoding the VIP peptide designed by optimizing degenerate codons for maximal protein expression in E. coli

<400> 2

gttcagaatg ctattcaagt actttttaac cgccatctgc ttacgaaggc gcgtataatt     60

atcggtaaac actgcatcac tatg     84

<210> 3
<211> 96
<212> DNA
<213> Artificial

<220>
<223> Artificially generated oligonucleotide (sense template) encoding the BH3 domain of the BID peptide with linker designed by optimizing degenerate codons for maximimal protein expression in E. coli

<400> 3

tctctgctga tcgccagacg catgccgaac ttcaacgaag atataatccg aaatattgcg     60

cgtcacctgg ctcaagtcgg tgattcaatg gaccgt     96

<210> 4
<211> 96
<212> DNA
<213> Artificial

<220>
<223> Artificially generated oligonucleotide (anti-sense template) encoding the BH3 domain of the BID peptide with linker designed by optimizing degenerate codons for maximal protein expression in E. coli

<400> 4

acggtccatt gaatcaccga cttgagccag gtgacgcgca atatttcgga ttatatcttc     60

gttgaagttc ggcatgcgtc tggcgatcag cagaga     96

<210> 5
<211> 210
<212> DNA
<213> Artificial

<220>
<223> Artificially amplified oligonucleotide encoding the fusion polypeptide with linker obtained by ligating the templates of seq IDS 1 to 4

<400> 5

```
catagtgatg cagtgtttac cgataattat acgcgccttc gtaagcagat ggcggttaaa      60
aagtacttga atagcattct gaacggatcc tctctgctga tcgccagacg catgccgaac     120
ttcaacgaag atataatccg aaatattgcg cgtcacctgg ctcaagtcgg tgattcaatg     180
gaccgtctcg agcaccacca ccaccaccac                                      210
```

<210> 6
<211> 32
<212> DNA
<213> Artificial

<220>
<223> Artificiallly generated oligonucleotide (sense primer) to amplify the VIP fragment using Seq IDS 1 and 2 as templates

<400> 6
aatcatatgc atagtgatgc agtgtttacc ga          32

<210> 7
<211> 33
<212> DNA
<213> Artificial

<220>
<223> Artificially generated oligonucleotide (anti-sense primer) to amplify the VIP fragment using Seq IDS 1 and 2 as templates

<400> 7
aatggatccg ttcagaatgc tattcaagta ctt          33

<210> 8
<211> 30
<212> DNA
<213> Artificial

<220>
<223> Artificially generated oligonucleotide (sense primer) to amplify the BH3 domain of the BID with linker fragment using Seq ID 3 and 4 as template

<400> 8
aatggatcct ctctgctgat cgccagacgc          30

<210> 9
<211> 32
<212> DNA
<213> Artificial

<220>
<223> Artificially generated oligonucleotide (sense primer) to amplify the BH3 domain of BID with linker fragment using Seq IDS 3 and 4 as templates

<400> 9
aatctcgaga cggtccattg aatcaccgac tt        32

<210> 10
<211> 71
<212> PRT
<213> Artificial

<220>
<223> This amino acid sequence was artificially generated using naturally occurring mature human VIP peptide produced in E.coli as fusion protein

<400> 10

```
Met His Ser Asp Ala Val Phe Thr Asp Asn Tyr Thr Arg Leu Arg Lys
1               5                   10                  15

Gln Met Ala Val Lys Lys Tyr Leu Asn Ser Ile Leu Asn Gly Ser Ser
            20                  25                  30

Leu Leu Ile Ala Arg Arg Met Pro Asn Phe Asn Glu Asp Ile Ile Arg
        35                  40                  45

Asn Ile Ala Arg His Leu Ala Gln Val Gly Asp Ser Met Asp Arg Leu
    50                  55                  60

Glu His His His His His His
65                  70
```

<210> 11
<211> 12
<212> PRT
<213> Artificial

<220>
<223> This sequence was artificially generated

<400> 11

```
Ser Leu Leu Ile Ala Arg Arg Met Pro Asn Phe Asn
1               5                   10
```

**Description**

The present invention relates to targeted fusion proteins for cancer therapy. In particular, the present invention relates to fusion proteins, A-B-C or C-B-A, consisting of three fused polypeptides, viz. A, B, and C, wherein the polypeptide A, is a neuropeptide, whose receptors are over expressed in tumor cells; polypeptide B is a peptide sequence that is recognized and cleaved by cancer specific protease; and polypeptide C is a bioactive protein or peptide, consisting of a BH3 domain of proapoptotic proteins. The present invention also relates to a pharmaceutical composition comprising the fusion proteins for use in targeting specific cell types in a cell mixture, and eliminating the target cells by inducing apoptosis.

The fusion proteins of the present invention specifically target cancer cells, over-expressing the neuropeptide receptors and are thereby useful as therapeutic agents for the treatment of cancers of various origin as well as a diagnostic marker for detection of tissues of origin of receptor cells.


**BACKGROUND OF THE INVENTION**

From cell division to programmed cell death, protein-protein interactions are a central regulatory feature of nearly all-biological processes in a living organism. In the recent past, molecular oncologists focused their studies primarily on the cellular pathways controlling proliferation. Neoplastic disease was typically envisaged as resulting from defects in these pathways leading to excess cell division. In fact, the ability to escape suicide (apoptosis) is a hallmark of most cancer cells and often correlates with tumor aggressiveness and resistance to traditional anticancer drug treatments [Schmitt C.A., Nature Rev. Cancer, 2003, 3, 286]. Consequently, there is an ongoing quest to develop new molecules that reactivate apoptosis in tumor cells by specifically targeting protein-protein interactions [Reed J.C., Cancer Cell, 2003, 3, 17.2]. Since tumors accumulate mutations which increase their resistance to natural apoptotic triggers, it is important to know how to manipulate the downstream apoptosis machinery in new, perhaps more direct, ways. Finding new bioactive proteins/peptides as therapeutic triggers, which induce tumor cell apoptosis in a manner independent of p53 (a critical tumor suppressor gene), has important clinical implications and remains a central focus in this area of apoptosis research. Similarly, shifting the balance of Bcl-2 family proteins in tumor cells may also trigger cell death. Incidentally, paclitaxel, a drug that acts p53-independently, is believed to exert its cytotoxic effects through its ability to phosphorylate and inhibit Bcl-2 downstream of p53 [Haldar S. et al., Proc Natl Acad Sci USA, 1995, 92, 4507]. Further, Bax, which is a Bcl-2 family protein has also been shown to enhance the cytotoxicity of chemotherapeutics like paclitaxel, vincristine and in a p53-independent fashion [Strobel T. et al. Proc Natl Acad Sci USA, 1996, 93, 14094]. Similarly, BID, which is also a Bcl-2 family protein has been shown to sensitize the apoptosis induced by 5-fluorouracil and doxorubicin in hepatocellular carcinoma [Miao J. et al., Int. J. Oncol., 2004, 25, 651]. These findings suggest that Bcl-2 family proteins are key players in the induction of apoptosis [Gross A. et al., Genes Dev., 1999, 13, 1899; Reed J., J. Clin. Oncol, 1999, 17, 2941].

The Bcl-2 family proteins consist of both proapoptotic and antiapoptotic members and are believed to regulate the cellular machinery [Abate-Shen C. et al., Genes Dev., 2001, 4, 2410; Lowe S.W: et al., Carcinogenesis, 2000, 21, 485]. In fact, it is logical to hypothesize that suppression of antiapoptotic and/or activation of proapoptotic members of the Bcl-2 protein family could significantly enhance the induction of apoptosis and therefore increase the efficacy of therapy.

The Bcl-2 family is characterized by specific regions of homology termed Bcl-2 homology (BH1, BH2, BH3, BH4) domains. These domains are critical to the functions of these proteins, including their impact on cell survival and their ability to interact.with other family members and regulatory proteins [Abate-Shen C. et al., Genes Dev., 2001, 4, 2410; Johnson A.L, Endocrinology, 1999, 140, 5465]. It was shown that the BH3 domain of proapoptotic proteins of this family is specifically responsible for the induction of apoptosis [Lowe S.W et al., Carcinogenesis, 2000, 21, 485; Johnson A.L, Endocrinology, 1999, 140, 5465; Cosulich S.C. et al., Curr. Biol, 1997, 7, 913]. Furthermore, expression of small-truncated derivatives of BAK protein containing the BH3 domain was sufficient for its apoptotic activity [Lutz RJ. Biochem. Sci. Trans, 2000, 28, Part 2-51]. Moreover, it was found that short synthetic peptides, corresponding to the BH3 domain sequence when fused to luteinizing hormone releasing hormone (LHRH) enhances the uptake of these peptides [Minko T. et al., Clin. Outcomes, 2001, 3, 48; Dharap S.S. et al., Pharm Res, 2003, 20, 889]. However, the practical use of the BH3 peptide is limited by its low permeation into cancer cells and therefore requires a suitable intracellular delivery system. In this regard a number of intracellular delivery systems are in vogue like polyarginine or polylysine chains, Tat peptides, antennapedia peptides etc [Schimmer, A.D. et al., Cell Death Differentiation, 2001, 8, 725; Zhao M. et al., Med. Res. Rev, 2004, 24, 1; Wender P.A, et al., Proc. Natl. Acad. Sci USA, 2000, 97, 13003].

A proapoptotic member of the Bcl-2 family is BID, which has been shown to be cleaved by caspase 8 & then BID translocates to mitochondria facilitating the release of cytochrome c. Furthermore BID is also known to interact with both Bcl-2 and BAX. [Esposti M.D., Apoptosis, 2002, 7(5),433-40]. BID is also phosphorylated following induction of double-strand breaks in DNA. DNA damage signals the activation of ATM and ATR kinases, which in turn either causes cell cycle arrest and DNA repair or apoptosis [Kamer I, et al., Cell, 2005, 122(4), 593-603]. As BID, with other members of the Bcl-2 family play key role in the apoptosis, shifting the balance of these molecules in tumor cells to induce the cell death may have a great potential in the cancer therapy.

The stapled peptides of BH3 domain of BID peptide (SAHB) were helical, protease-resistant, and cell-permeable, with increased affinity to Bcl-2 pockets. SAHB (2-10$\mu$M) specifically activated the apoptotic pathway to kill leukemia cells. SAHB (10 mg/kg) inhibited the growth of human leukemia xenografts in vivo [Walensky L. et al., Science, 2004, 305, 1466-70].

From the foregoing, it would be amply evident that development of agents, which not only are specific and selective in targeting the cancer cells but also can cause apoptosis, is the need of the hour and remains a major goal in the treatment of cancer. Such an agent must achieve high cell permeability and retention by the specific cell population. Few such targeted agents, which have been reported, are:

i) Holinger et al., [J. Biol. Chem., 1999, 274, 13298-13304] found that a fusion peptide of the BH3 domain of BAK and the antennapedia protein internalization domain could induce apoptosis in HeLa cells. The over-expression of Bcl-XL could prevent this apoptotic response, but the Ant-BAK BH3 peptide was still able to re-sensitize the Bcl-XL-over-expressing cells to Fas-induced apoptosis. This Ant-BAK BH3 peptide was also found to induce substantial cell death in erythrocyte cultures [Walsh M .et al., Blood, 2002, 99, 3439-3448].

It is, however, found that the Ant-BAK BH3 peptide disclosed by Holinger et al. suffers from a limitation in that it induces substantial cell death in erythrocyte cultures, thereby, emphasizing the need for meticulous toxicity testing before any pro-apoptotic peptide is considered for clinical testing [Walsh M. et al., Blood, 2002, 99, 3439-3448].

ii) Wang et al., [Cancer Res, 2000, 60, 1498-1502] have reported that a peptide of the proapoptotic protein, viz. Bad fused to decanoic acid and a cell-permeable moiety, (cpm)-1285 could enter HL-60 tumor cells, bind Bcl-2, and induce apoptosis, but had minimal effect on normal human peripheral blood lymphocytes. Wang et al, further mention that cpm-1285 slowed the growth of human myeloid leukemia in severe combined immuno-deficient mice, but caused no gross signs of organ toxicity in normal C57BL/6J mice.

The fusion peptides disclosed by Wang et al., has minimal effect on normal human peripheral blood lymphocytes, however in the absence of any targeting moiety, it would probably be toxic to normal cells.

iii) Ellerby et al. [Nature Med, 1999, 1-32, 1038] have designed short peptides composed of two functional domains, one comprising of a tumor blood vessel 'homing' motif, a cyclic 'CNGRC' peptide or a doubly-cyclic 'ACDCRGDCFC' peptide and the other comprising a programmed cell death-inducing sequence, the synthetic alpha-helical 14-amino acid peptide "KLAKLAKKLAKLAK" These peptides were selectively toxic to angiogenic endothelial cells and showed anti-cancer activity in mice. A novel peptide, DP1, comprised of a protein transduction domain (PTD), PTD-5, fused to an antimicrobial peptide, (KLAKLAK)$_2$, was able to trigger rapid apoptosis in a variety of cell lines in vitro, including MCA205 murine fibrosarcomas and human head and neck tumors [Mai et al., Cancer Res, 2001, 7709-7712].

However, these fusion peptides disclosed by Ellerby et al lack specificity towards cancer cells and should be internalized into mammalian cells to induce cell death and apoptosis by disrupting the negatively charged mitochondria.

iv) Mouse BAD gene was fused to the gene for the translocation and receptor-binding domains of diptheria toxin. The resulting fusion protein showed cytotoxicity to human glioma cells in a dose-dependent manner [Ichinose et al., Cancer Res, 2002, 62,1433-1483].

This fusion peptide disclosed by Ichinose et al., however lacks cancer specific cell targeting domain and may lead to non-selective apoptosis.

v) Thornburn et al. (Clinical Cancer Res., 2003, 9, 861-865] designed a targeted fusion toxin consisting of diphtheria toxin fused to granulocyte macrophage colony stimulating factor (GMCSF) (DT$_{388}$-GMCSF) to selectively kill acute myeloid leukemia cells. Immunotoxins have been constructed to specifically target and destroy cells expressing such surface molecules, among them cancer cells and cells involved in disorders of the immune system. The targeting moiety of the immunotoxins consists of whole antibodies or antigen-binding domains or growth factors and cytokines. These molecules are generally fused to various mutant forms of bacterial or plant toxin molecules to destroy the unwanted cells [Brinkmann U. et al., Biochim. Biophys Acta., 1994, 1198, 27-45].

The major drawback in the clinical application of these molecules is the human immune response they elicit, mainly toward the toxin moiety. Bacterial toxins like Pseudomonas exotoxin A (PE) and diphtheria toxin are highly immunogenic and cannot be humanized by standard techniques.

vi) Aqeilan et al. [FEBS letters, 1999, 271-276; US 6,645,490] have developed a novel chimeric protein, consisting of IL-2 as the targeting moiety, which specifically targets IL2R-expressing cells with the full-length human BAX protein, to induce IL2R specific apoptosis.

The main drawback of IL2-BAX chimeric protein disclosed by Aqeilan et al., is that IL2-BAX selectively kills the tumor cells which are activated lymphocytes but may not be useful to treat a variety of tumors which lack IL-2 receptors. Furthermore, the chimeric protein encompasses a full length BAX protein, which makes the protein large and difficult in its isolation, solubilization and refolding from inclusion bodies. Partially refolded IL2-BAX may not be active in killing the cells by apoptosis, as its BH3 domain should be helical for activity.

vii) Dharap et al. [Pharm Res, 2003, 20, 889] linked the luteinizing hormone-releasing hormone (LHRH) with a synthetic Bcl-2 homology 3 (BH3) domain peptide as an apoptosis inducer and a suppressor of antiapoptotic cellular defense, to target cancer cells, which express LHRH-receptors (ovarian, breast, prostate). The drawback of LHRH receptors is that they are over expressed in ovarian, breast and prostate cancers only. LHRH-BH3 domain fusion protein may not be useful on a variety of other tumors of different origin.

From the abovementioned reports, it would be obvious that the fusion proteins reported till now suffer from any one or more of the following limitations, viz.

a) Lack specificity towards cancer cells;
b) Elicit human immune response and hence cannot be humanized by standard techniques;
c) Lack applicability to treat variety of tumors of different origin;
d) Encompass full-length fusion partners, which makes the protein large and difficult in its isolation, solubilisation and refolding from inclusion bodies. Partially refolded BH3 domain containing fusion proteins may not be active in killing the cells by apoptosis, as the BH3 domain(s) should be helical for its activity.

The present invention is a step forward in this direction and provides a fusion peptide, which is found to be specific for most of the cancer cells.

## OBJECTS OF THE INVENTION

An object of the present invention is to provide fusion proteins, which specifically target cancer cells, over-expressing the desired receptors.

Another object of the present invention is to provide fusion proteins, which eliminate the target cancer cells by inducing apoptosis.

A further object of the present invention is to provide fusion proteins useful as therapeutic agents for treatment of cancer of various origins.

Still further object of the present invention is to provide fusion proteins useful as diagnostic markers for detection of tissues of origin of receptor cells.

## SUMMARY OF THE INVENTION

In accordance with the above objects, the present inventors have found that a novel fusion protein comprising of three fused polypeptides specifically targets cancer cells over-expressing the neuropeptide receptors and are thereby useful as therapeutic agents for the treatment of cancers of various origins as well as a diagnostic marker for detection of tissues having expression of receptors to which it is targeted.

The novel fusion proteins consist of three fused polypeptides, viz. A, B, and C, fused as A-B-C or C-B-A, wherein the polypeptide A is a neuropeptide selected from vasoactive intestinal peptide (VIP), somatostatin (SST), Gastrin releasing peptide (GST), Bombesin (BOM), or Substance P (Sub P), whose receptors are over expressed in tumor cells; polypeptide B is a linker that is recognized and cleaved by cancer specific protease having the amino acid sequence S L L I A R R M P N F N (SEQ ID No. 11); and polypeptide C consists of a BH3 domain of a pro-apoptotic protein selected from Bak, Bax, Bim, Bid and Bad.

Polypeptide A provides cell-specific targeting properties to the fusion protein, while the cleavage of the linker (polypeptide B) by cancer specific proteases releases Polypeptide C i.e. apoptosis-inducing moiety and upon entry into a target cell leads to programmed cell death, which is then eliminated by phagocytosis, thus avoiding any tissue or systemic response. Hence, Polypeptide A transports polypeptide C into the tumor cells and aids in modulation of desired activity. The desired activity could be cell viability, cell death, metabolism, stasis, apoptosis etc.

It is preferred that the fusion proteins, A-B-C or C-B-A of the invention be produced by recombinant expression of a fusion polynucleotide.

Vasoactive intestinal peptide (VIP) is a 28-amino acid neuropeptide, which was first isolated from the porcine intestine. There are two commonly known subtypes of VIP receptors, viz. VPAC 1 and VPAC 2. It has been found that tumors expressing VPAC1 receptors include the most frequently occurring malignant epithelial neoplasms, such as cancers of the lung, stomach, colon, rectum, breast, prostate, pancreatic ducts, liver, and urinary bladder [Reubi J.C, et al., Cancer Res, 2000, 60, 3105-3112]. In fact, VPAC1 mRNA was identified in many tumor cell lines, such as lung and breast cancers [Vaudry D., et al., Pharmacol Rev., 2000, 52, 269-324]. In contrast to this ubiquitous expression of VPAC1 receptors in most human tumors, a predominance of VPAC2 receptors was found in only few tumors. Several different human tumor types express predominantly PAC1 receptors, in particular tumors originating from the neuronal and endocrine systems; this includes glial tumors (astrocytomas, glioblastomas, oligodendrogliomas), neuroblastomas, as well as various pituitary adenomas (especially GH-secreting and nonfunctioning adenomas, but not prolactinomas), as described previously by Robberecht et al., [Robberecht P. et al., **1994**, 15, 661-665; Robberecht P et al., J Clin Endocrinol Metab., 1993, 77, 1235-1239; Vertongen P., et al., J Cell Physiol., 1996, 167, 36-46]and by Oka et al. [Oka H. et al., Am. J. Pathol., 1998, 153, 1787-1796]. More recent data have shown that most catecholamine-secreting tumors, including both pheochromocytomas and paragangliomas, appear to express predominantly PAC1 receptors [Reubi J.C et al., Cancer Res., 2000, 60, 3105-3112]. Moreover, many endometrial carcinomas also have PAC1 receptors [Reubi J.C et al., Cancer Res., 2000, 60, 3105-3112].

Somatostatin is another neuropeptide which consists of a family of a 14-amino-acid (somatostatin-14) and a 28-amino-acid (somatostatin-28) peptide. It appears in several organ systems, such as the central nervous system, the hypothalamopituitary system, the gastrointestinal tract, the exocrine and endocrine pancreas, and the immune system. Moreover, somatostatin plays a role in cancer: in many animal tumor models and cultured tumor cell lines, somatostatin and somatostatin analogs inhibit tumor growth [Schally A.V, Cancer Res., 1988, 48, 6977-6985]. To date, five human somatostatin receptor subtypes (sst1, sst2, sst3, sst4; and sst5) have been cloned and partially characterized [Reisine T. et al., Endocr Rev., 1995, 16, 427-442; Hoyer D. et al., Trends Pharmacol Sci., 1995, 16, 86-88].

Bombesin and GRP, members of neuropeptide family are brain-gut peptides, play an important role in cancer [Cuttitta F. et al., Nature, 1985,316, 823-825], in addition to their physiological function. Bombesin is a 14-amino-acid peptide present in amphibian tissues, whereas GRP, its human counterpart, consists of 27 amino acids. GRP and bombesin differ by only one of the 10 carboxy-terminal residues. Bombesin and GRP mediate their actions through membrane-bound, G protein coupled receptors, which include at least four different subtypes, namely the neuromedin B receptor subtype (BB1), the GRP receptor subtype (BB2), the BB3 and BB4 subtypes [Wada E et al., Neuron, 1991, 6, 421-430; Fathi Z. et al., J Biol Chem, 1993, 268, 5979-5984; Nagalla SR et al., Proc Natl Acad Sci USA, 1995, 92, 6205-6209].

Substance P is a neuropeptide involved in a variety of functions of the central and peripheral nervous systems, including pain perception and vasodilatation [Hokfelt T et al., J Intern Med, 2001, 249, 27-40]. Substance P receptor subtypes include the NK1, NK2, and NK3 receptor. NK1 receptor subtype is quite frequently expressed in glial tumors, in particular in poorly differentiated glioblastomas, but can also be detected in medullary thyroid carcinomas, small cell lung cancers, pancreatic as well as breast cancers [Hennig I.M. et al., Int J Cancer, 1995, 61, 786-792; Friess H et al., Lab Invest 2003, 83, 731-742].

LHRH is hypothalamic hormone acting primarily at the pituitary level to stimulate LH secretion. LHRH receptors were found in other tumors as well, including prostatic, endometrial, and ovarian carcinomas [Irmer G. et al., Cancer Res, 1995, 55,817-822; Emons G. et al., Hum Reprod, 1994, 9, 1364-1379; Halmos G. et al., J Urol, 163, 623-629; Straub B. et al., Clin Cancer Res, 2001, 7, 2340-2343] and were characterized as belonging to the low-affinity GnRH-II subtype (Grundker C et al., J Clin Endocrinol Metab, 2002, 87, 1427-1430].

In particular, it has been found that amongst the polypeptides A mentioned hereinbefore, VIP is the choice as the targeting moiety as majority of tumors over-express VPAC1 receptors, which include the most frequently occurring malignant epithelial neoplasms, such as cancers of the lung, stomach, colon, rectum, breast, prostate, pancreatic ducts, liver, and urinary bladder [Reubi J.C et al., Cancer Res., 2000, 60, 3105-3112]. A large number of cells surface receptors have been evaluated for specific delivery of drugs/peptide into cancer cells [Vyas S.P.et al., Crit. Rev.Ther. Drug Carrier Syst, 2001, 18, 1-76]. Peptide hormone receptors remain very popular targets for targeted drug/peptide delivery [Langer M. et al., Curr. Med. Chem. Anti-Canc. Agents, 2001, 1, 71-93]. All neuropeptide receptors are G-Protein coupled receptors (GPCR) and undergo endocytosis with subsequent degradation of the ligands in lysosomes. But most of the primary human tumors express vasoactive intestinal peptide receptors (VIP receptors) at high incidence [Reubi J.C. et al., Cancer Res., 1996, 56, 1922-1931; Reubi J.C, J Nucl. Med., 1995, 36, 1846-1853].

An additional specificity is the utility of cancer specific proteases site in the linker between targeting moiety and the apoptotic moiety. Furthermore, the linker was cleavable by cathepsin, which is expressed in the lysosome, thus on receptor internalization the designed protein will be cleaved at the desired site releasing the apoptotic domain. The BH3 domains of the Bcl2 members Bak, Bax, Bim, Bid and Bad were chosen as the apoptotic moiety as they are helical and sufficient to induce apoptosis when compared to taking the whole BH3 domain containing protein.

The recombinant fusion protein of the present invention may be used to treat diseases including various forms of cancers such as T- and B-cell lymphoproliferative diseases, ovarian cancer, pancreatic cancer, head and neck cancer, squamous cell carcinomas, gastrointestinal cancer, breast cancer, prostate cancer, and non-small cell lung cancer.

**BRIEF DESCRIPTION OF THE ACCOMPANYING DRAWINGS**

The invention will be better understood with reference to the drawings which are as under:

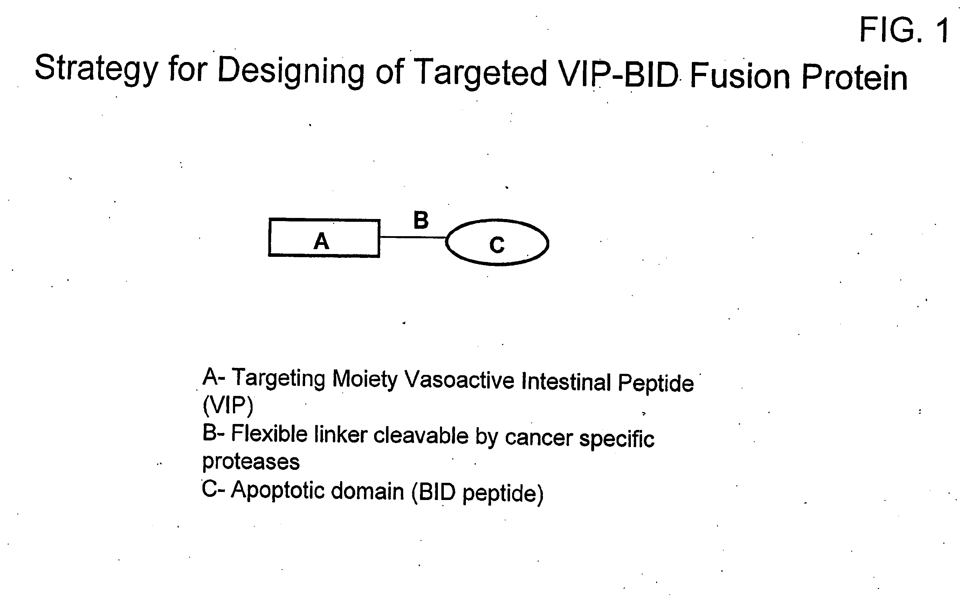

FIG. 1          Design of the fusion partners and their linkage by peptide linker.

FIG. 2          Nucleotide and the amino acid sequence of the fusion protein (SEQ ID NOs: 5, 10 of Table I).

FIG. 3          Cloning strategy used to generate the p-fusion protein Construct (SEQ ID NO: 5 of Table I).

FIG. 4          PCR amplification of individual fragments, viz. VIP and Linker BH3 BID.

FIG. 5          Ligation of individual fragments, viz. VIP and Linker BH3 BID.

FIG. 6          Fusion insert fallout from p-fusion-transformed clones.

FIG. 7          Induction of p-fusion protein-transformed clones using IPTG.

FIG. 8          Chromatogram of the fusion protein purified through nickel affinity

FIG. 9          chromatography. Eluted fraction analysed by SDS-PAGE, showing the pure fusion protein.

FIG. 10         Immuno-blots probed with the antibodies directed against VIP, BID and

FIG. 11         Poly His tag. Mass spectra of pure fusion protein (SEQ ID NO: 10 of Table I).

FIG. 12         Circular dichorism (CD) spectra of pure fusion protein in presence of

FIG. 13         different concentration of Trifluoroethanol (TFE), Sodiumdodecyl Sulphate (SDS). Percent viability of cancer cell lines following treatment with fusion

FIG. 14 (a)     protein for 24h as quantified by MTT assay. Percent viability of HT29 cells following treatment with 10 $\mu$M fusion

FIG. 14 (b)     protein in presence or absence of different concentrations of VIP (10-28) fragment for 24 Hrs, as quantified by MTT assay. Percent viability of HT29 cells following treatment with 5 $\mu$M fusion

FIG. 15         protein in presence or absence of different concentrations of VIP (10-28) fragment for 24 Hrs, as quantified by MTT assay. Immunocytochemical analysis of fusion protein binding to VPAC 1

FIG. 15 (a)     receptors expressed on A549 cells. Control cells.

FIG. 15 (b)     Cells treated with anti-VPAC 1.

FIG. 15 (c)     Cells treated with fusion protein followed by anti-BID.

FIG. 15 (d)     Cells pretreated with VIP antagonist VIP (10-28) fragment followed by anti-VPAC 1.

FIG. 15 (e)     Cells pretreated with VIP antagonist VIP (10-28) fragment followed by fusion protein and anti-BID.

FIG. 15 (f)     Cells pretreated with fusion protein followed by anti-VPAC.

FIG. 16         The UV chromatograms of fusion protein digestion with cathepsin D as a function of time.

FIG. 17(a-c)    Total ion chromatograms and deconvoluted spectra of the major peaks obtained by LC-MS after 30min digestion of fusion protein with cathepsin D.

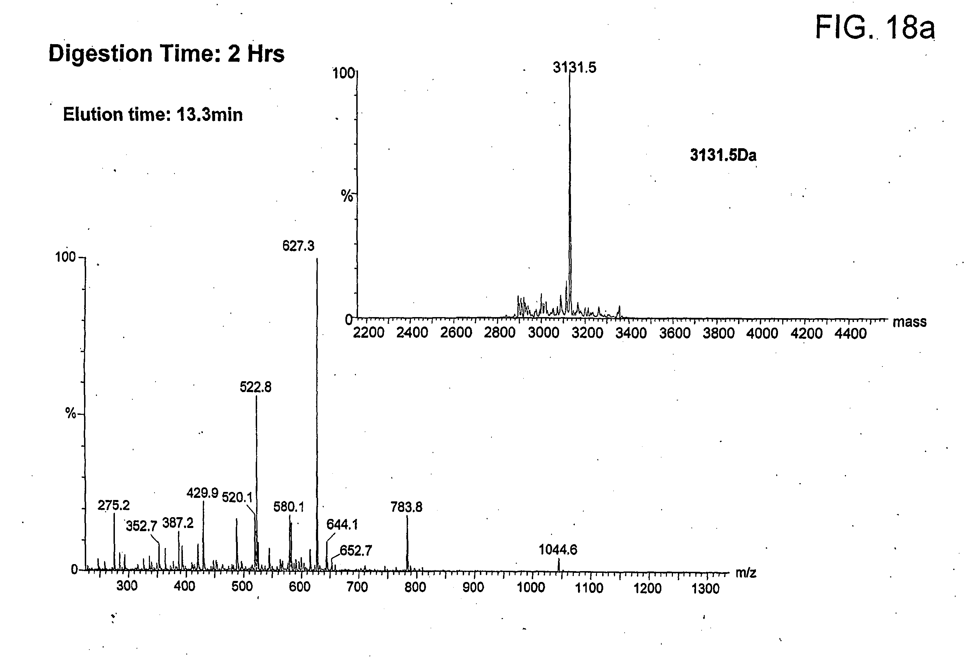

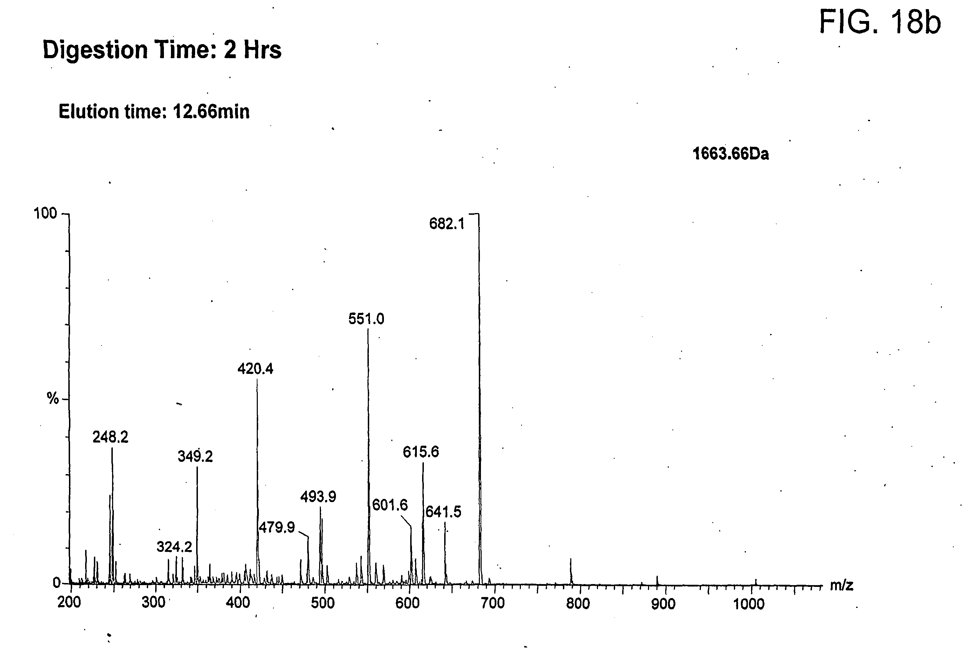

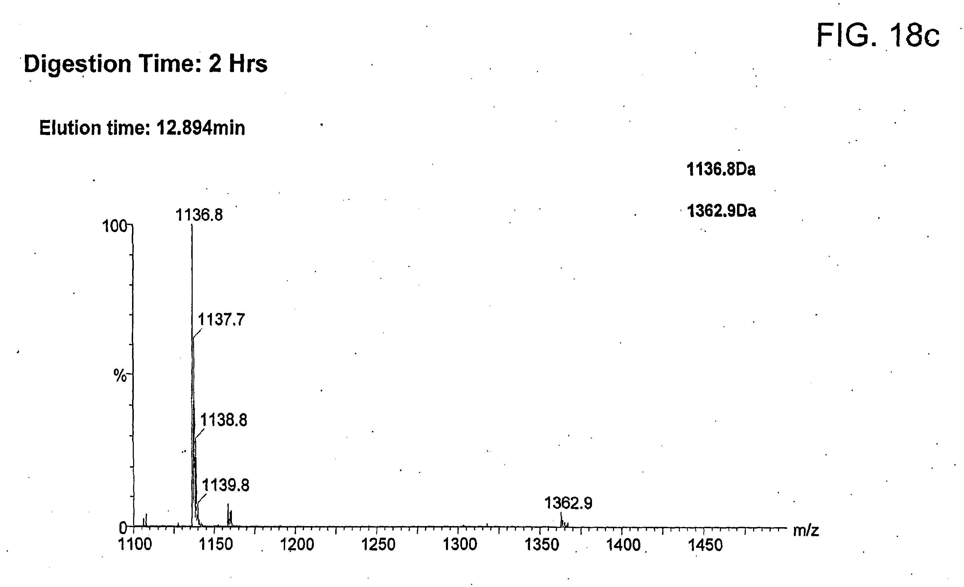

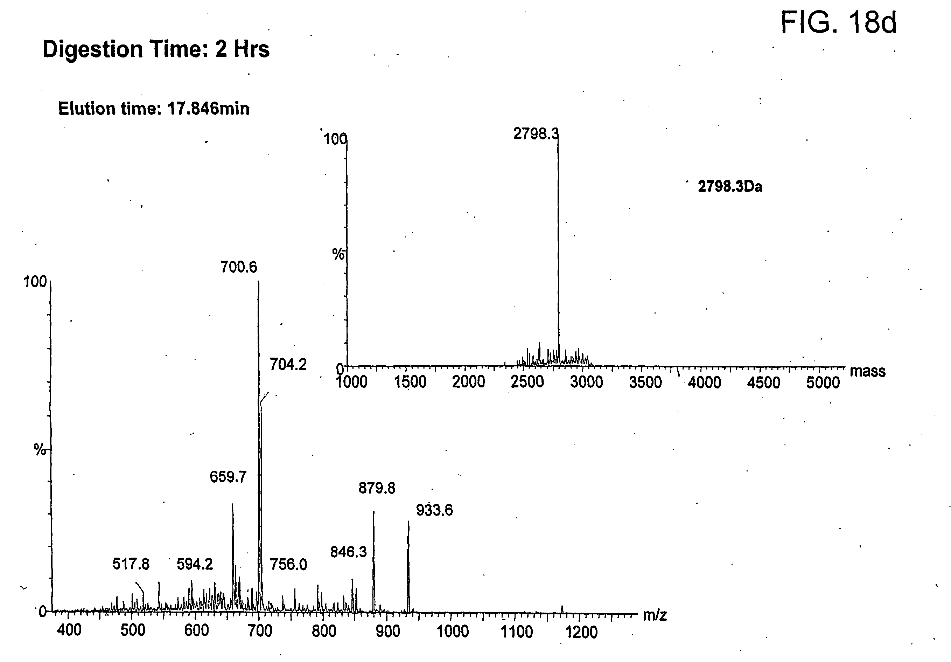

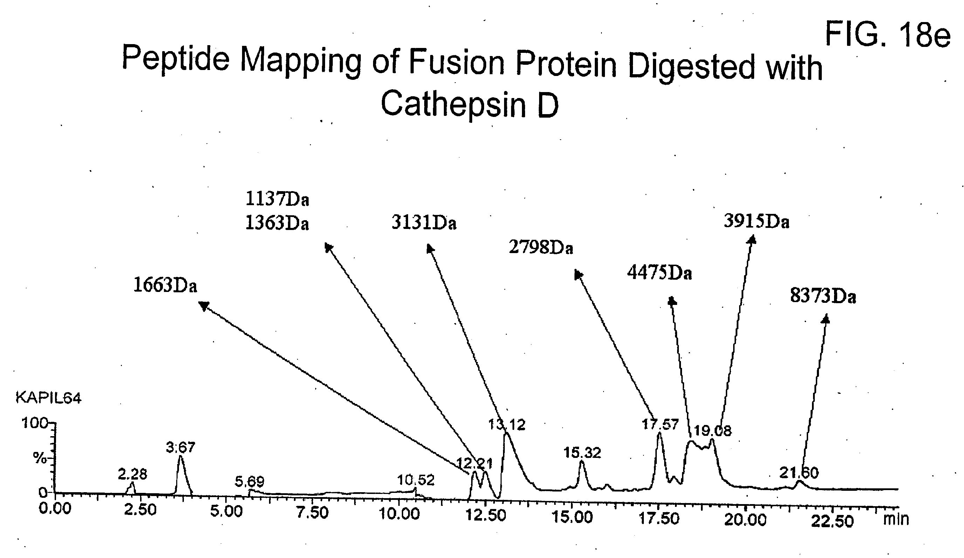

FIG. 18(a-e)    The UV chromatograms, the total ion chromatograms and deconvoluted

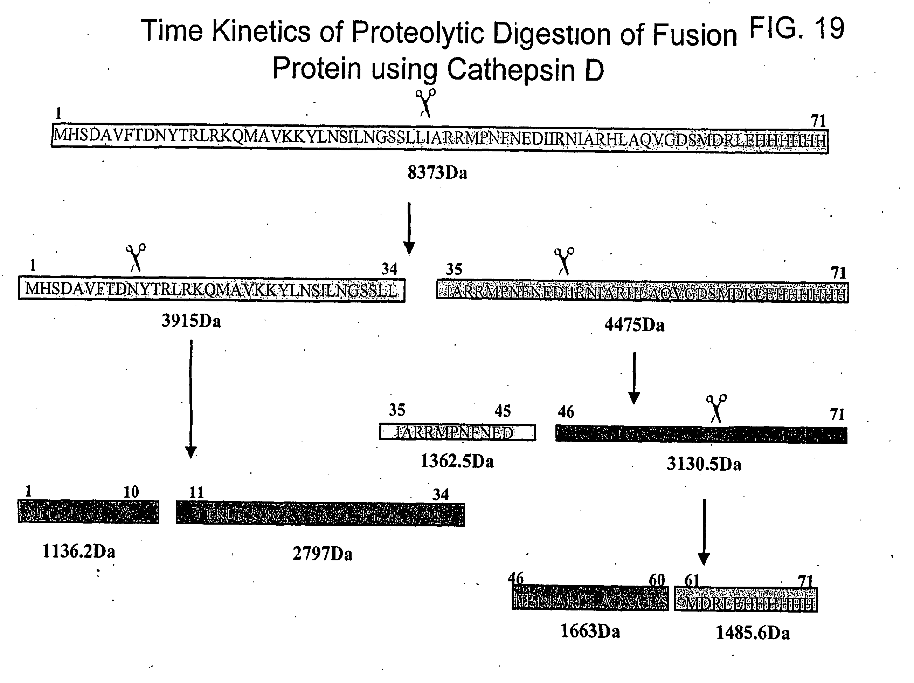

FIG. 19         spectra of the major peaks obtained by LC-MS after 2Hour digestion of fusion protein with cathepsin D. Proposed digestion profile of fusion protein with cathepsin D as a function

FIG. 20 (a)     of time. Control A549 cells stained with JCl showing red (health) fluorescence and green (apoptotic) fluorescence.

FIG. 20 (b)     A549 cells treated with fusion protein at a concentration of 125mg/ml and

FIG. 20 (c)     A549 cells treated with fusion protein at a concentration of 250mg/ml and stained with JCl showing apoptotic cells (green) fluorescence.

FIG. 20 (d)     stained with JCl showing apoptotic cells (green) fluorescence. Fluorescent intensity as a measure of mitochondrial membrane potential in

FIG. 20 (e)     healthy and apoptotic cells after fusion protein treatment. Plot of decrease in the ratio of red to green fluorescent intensity after fusion protein treatment.

## DETAILED DESCRIPTION

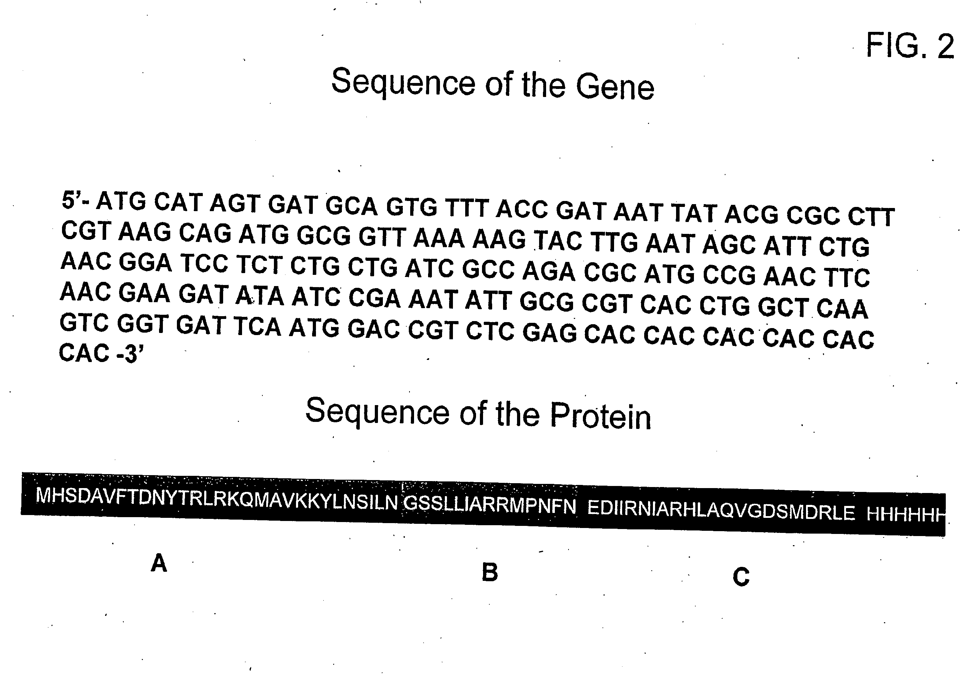

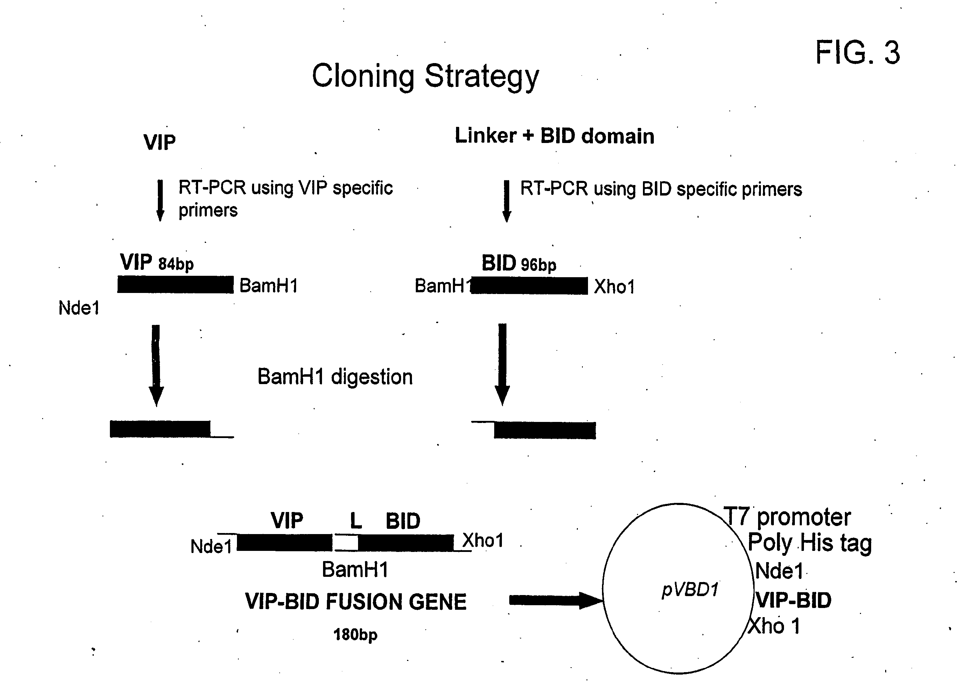

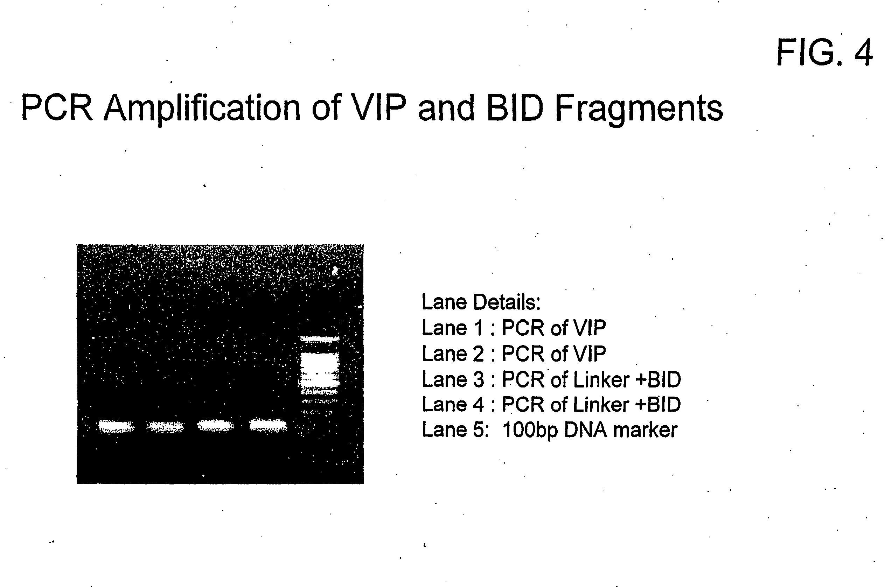

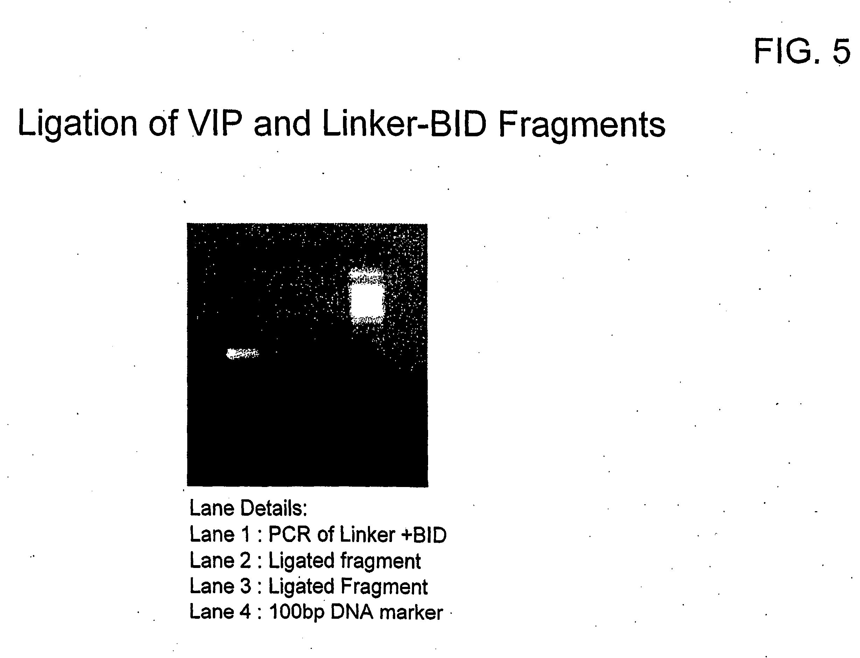

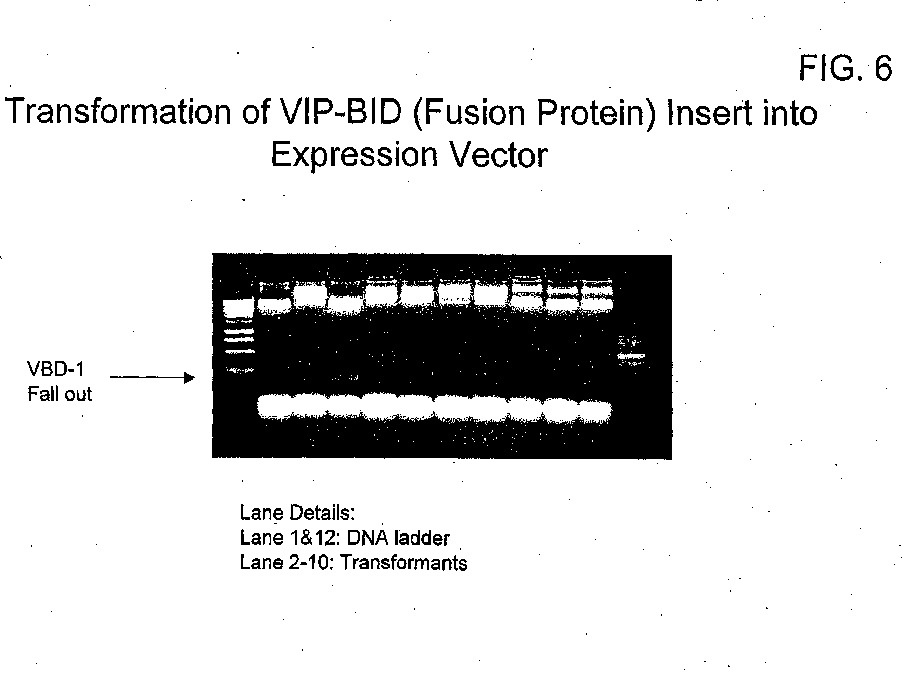

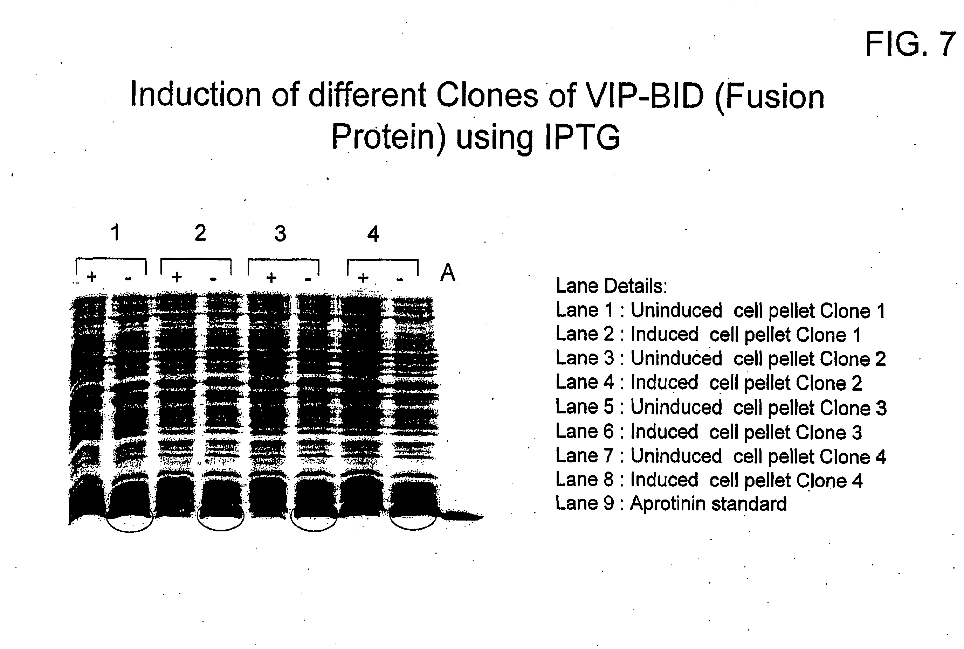

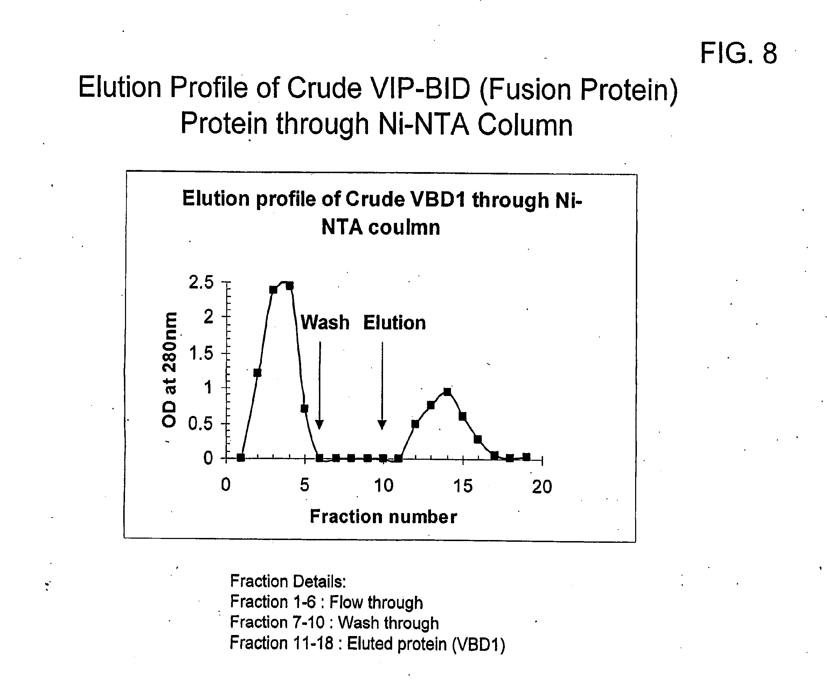

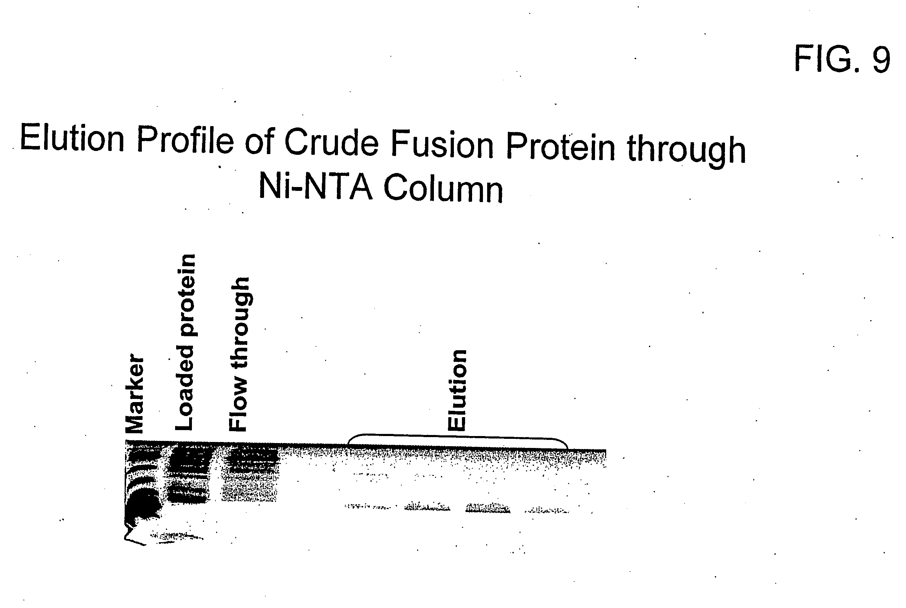

An example of the designed fusion gene of the present invention encoded a 71 amino acid polypeptide (SEQ ID: 10, FIG. 2 of Table I). The strategy used for cloning of the synthetic gene is given in FIG. 3. Oligonucleotides corresponding to VIP peptide and BID BH3 domain were either synthesized or amplified from the human cancer cell lines over expressing these peptides/proteins and the desired linker was introduced using suitable primers (FIG. 4). The oligonucleotide fragments were then ligated and amplified using PCR (FIG. 5) and the ligated product was then inserted into a pET22b+ expression vector under the control of the T7 promoter to yield an expression plasmid encoding fusion protein (FIG. 6). The protein was expressed in *E. coli* and the expression was induced by IPTG (FIG. 7). The fusion protein was isolated and purified by nickel affinity chromatography using an imidazole gradient (FIGs. 8 and 9).

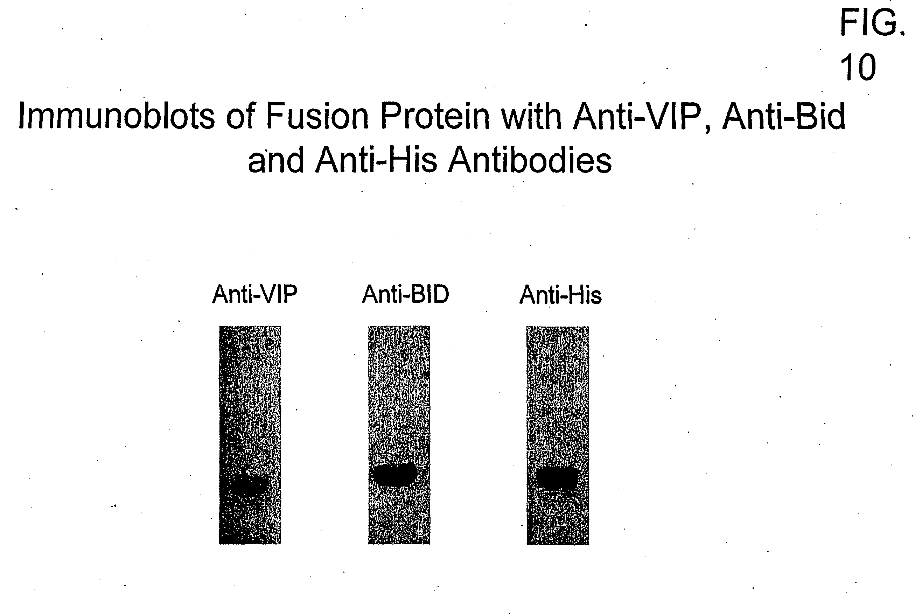

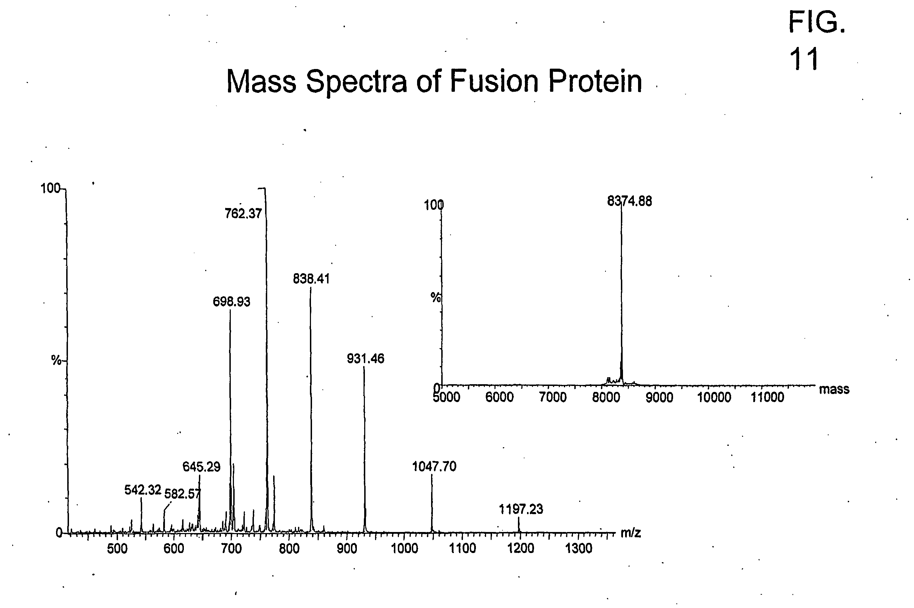

The fusion protein was further characterized by Western blot, using antibodies against VIP, BID and poly His tag. The fusion protein reacted with the antibodies to VIP, BID and Hexa His tag, confirming the cloning and production of in-frame full-length fusion protein (FIG. 10). Further, LC-electrospray ionization mass spectrometry of the protein gave a mass of 8374.8Da (expected mass of 8372.5Da), confirming the identity of the protein (FIG. 11).

To determine if the designed polypeptide had helical structure, which would be imperative for both the receptor binding by VIP and induction of apoptosis by BID, we recorded a circular dichrograph of fusion protein in presence of membrane-mimicking solvent, trifluoroethanol (TFE) and sodium dodecyl sulfate (SDS) [Carver J.A., et al., Eur. J.

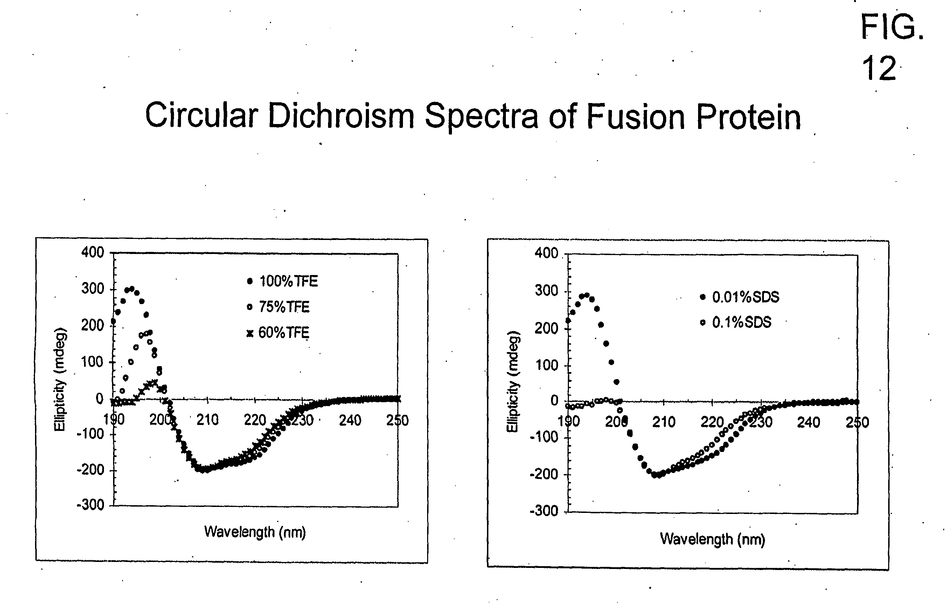

Biochem., 1990, 187, 645-650]. It was found that the synthetic gene was predominantly helical with characteristic minima at 208nm and 222nm, confirming that the designed protein retained its helical nature and therefore both targeting and cytotoxicity may be achieved with fusion protein (FIG. 12).

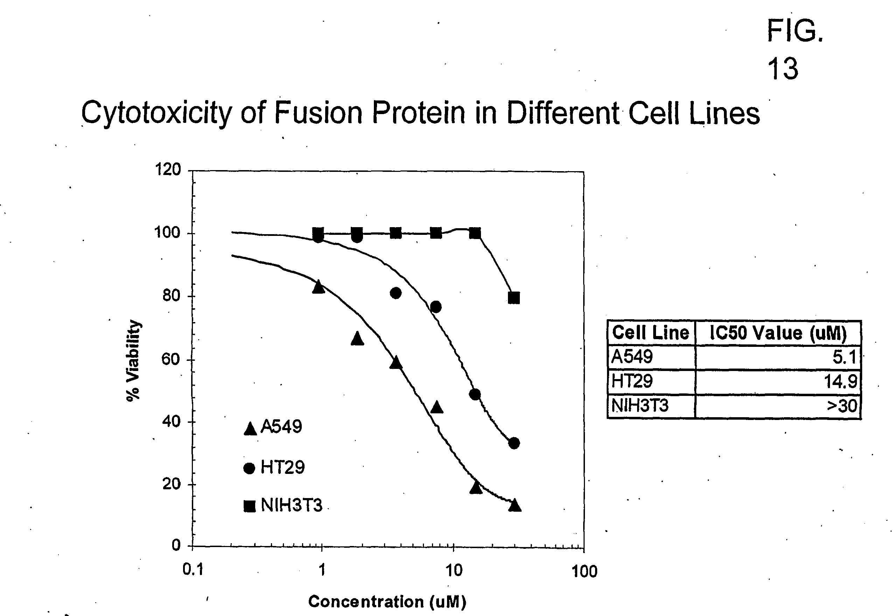

Subsequently, cytotoxicity of this protein was determined on cell lines over expressing the VIP receptor like colon carcinoma cell line, HT29 and non small cell lung carcinoma cell line, A549 and compared with a normal murine cell line, NIH3T3, by a quantitative MTT assay. As shown in FIG. 13, both the cell lines expressing VPAC1 receptors were responsive to the fusion protein in a dose dependent manner, with an $IC_{50}$ value of ~15$\mu$M and ~5$\mu$M for HT29 and A549 cell lines respectively. Different sensitivities of the two cell lines may be attributed to the differential expression of the VPAC1 receptors and the inherent sensitivity of the cell lines. Interestingly, the mouse fibroblast cell line, NIH3T3 was least sensitive with an $IC_{50}$ value of >30$\mu$M, suggesting that the cytotoxicity was definitely more pronounced in cell lines over expressing the VIP receptor, indicating the targeting of the polypeptide. This observation becomes quite interesting in light of earlier data of Letai et al who have shown that a BID peptide linked with a polyarginine tail had an $IC_{50}$ value of ~30$\mu$M in Jurkat cell line [Letai A. et al., Cancer Cell, 2002, 2, 183]. Similarly, Walensky et al. have shown as $IC_{50}$ value of BID peptide of around 10$\mu$M whereas, a modified (stapled) BID peptide gave an $IC_{50}$ value of between 2.2$\mu$M to 10.2$\mu$M on number of leukemia cell lines but had no specificity [Walensky, L.D.et al., Science, 2004, 305, 1466], suggesting the increased targeting potency of the designed polypeptide.

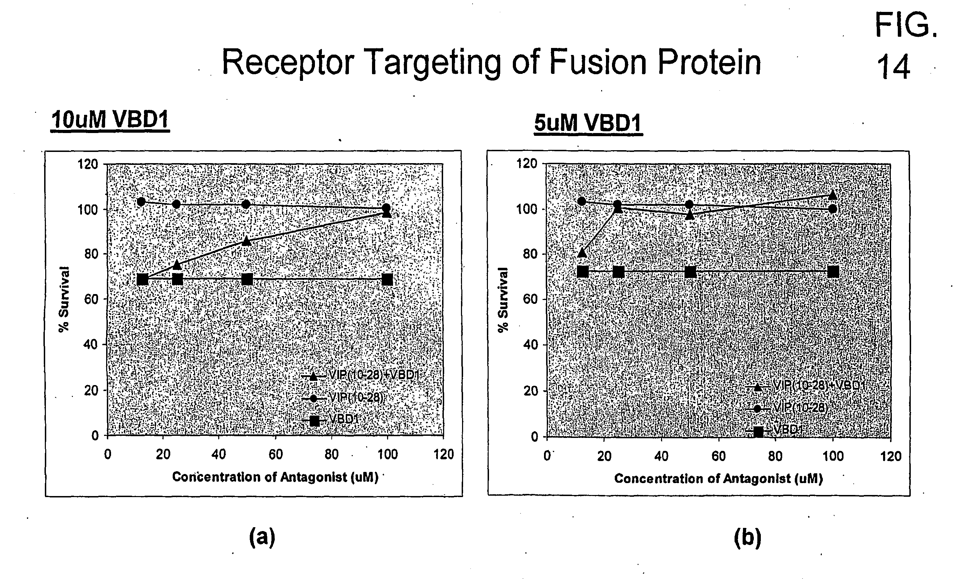

To further confirm the receptor mediated targeting of the protein, HT29 cells were pre-incubated with varying concentrations of VIP antagonist, VIP (10-28) peptide [Gozes I., et al., Cell Mol. Neurobiol., 1995, 15, 675] for 30 minutes followed by treatment with 10$\mu$M of fusion protein. It was found that there was a decrease in the cytotoxicity with an increasing concentration of the antagonist, with a complete loss of cytotoxicity at 100$\mu$M of VIP (10-28) peptide (FIG. 14a). A similar observation was made when the cells were treated with 5$\mu$M of fusion protein, although this time the receptors were saturated by 25$\mu$M concentration of the antagonist (FIG. 14b). This observation clearly indicates that there is a competition for the VPAC-1 receptors among fusion protein and the VIP (10-28) peptide antagonist with a bias towards the antagonist in form of pre-incubation of the cells with it, there is a loss in the cytotoxic activity of the fusion protein.

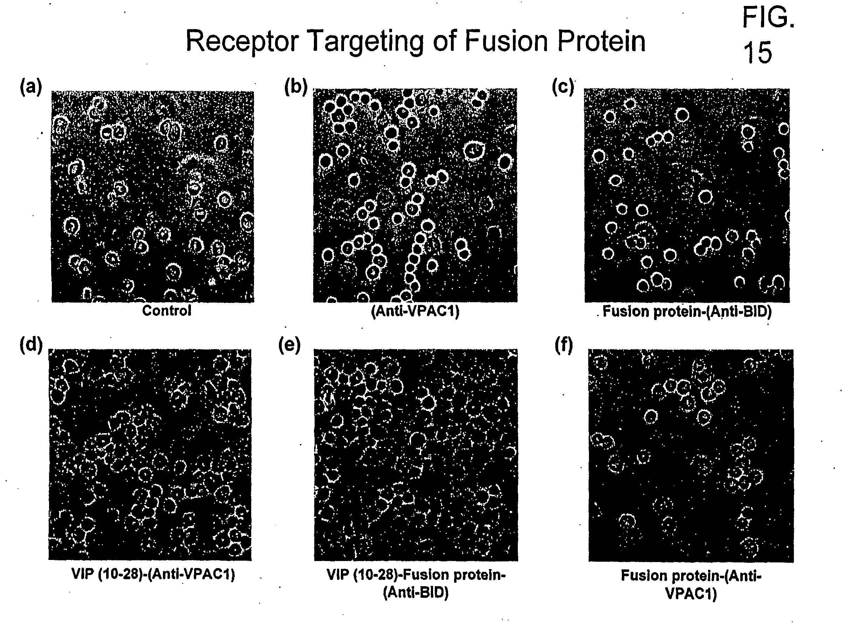

Immunostaining studies with the VPAC-1 antibody also revealed that the receptors were overexpressed in the A549 cell line (FIG. 15a, b). Similarly, when the cells were treated with fusion protein and immuno-stained with anti-BID. It was found that there was a localization of the protein on the cells (FIG. 15c) but when the cells were pre-treated with either VIP antagonist, VIP (10-28) or the fusion protein VPAC-1 receptors were not available to the anti VPAC-1, as seen with a significant loss in the immunostained cell population (FIG. 15d, f). A similar observation was made when A549 cells were pre-treated with VIP antagonist, VIP (10-28) followed by fusion protein and immunostaining with anti-BID (FIG. 15e). These observations conclusively suggest the receptor mediated targeting of the fusion protein to the tumor cells.

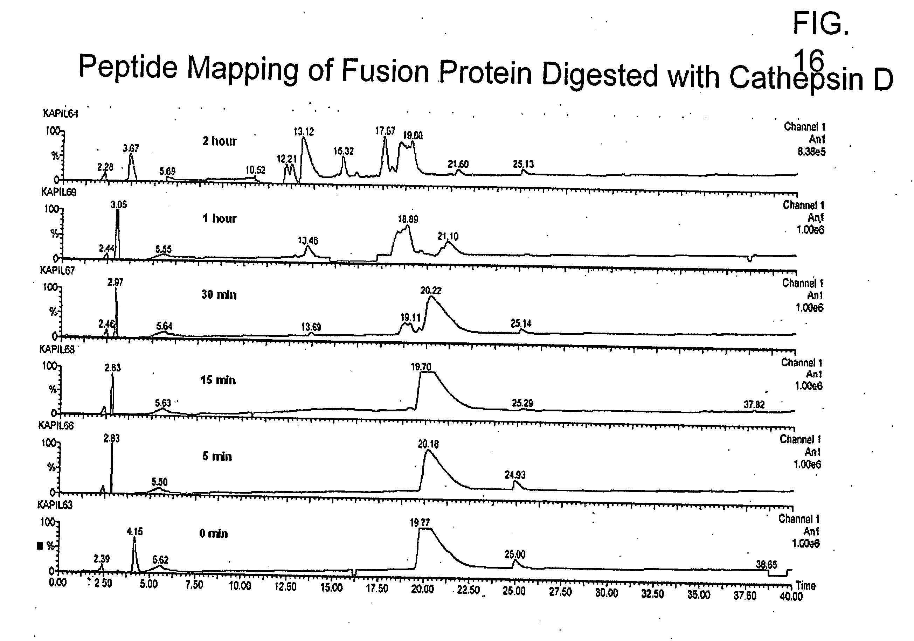

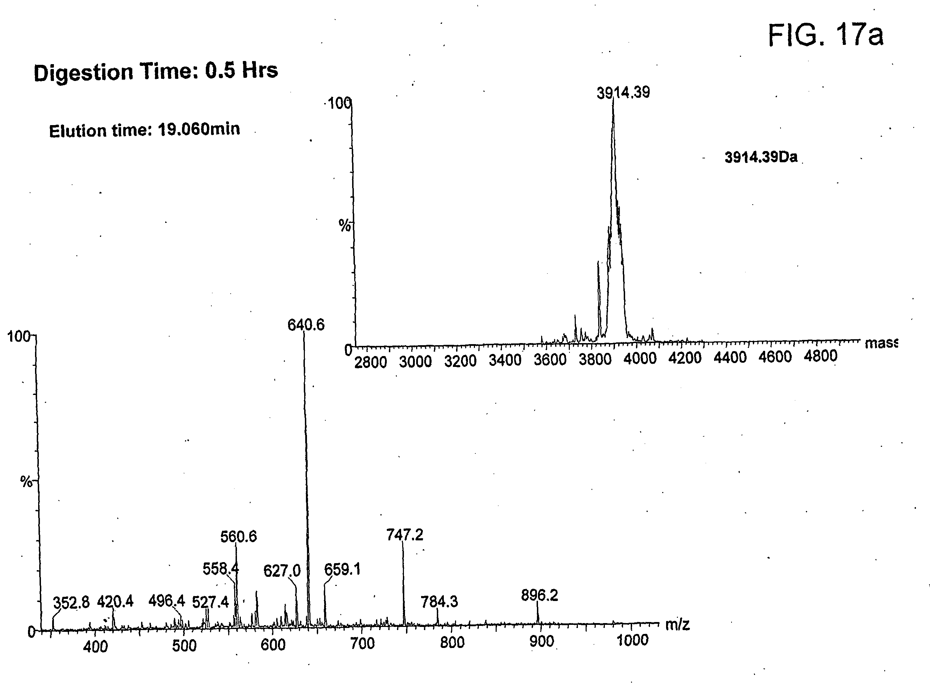

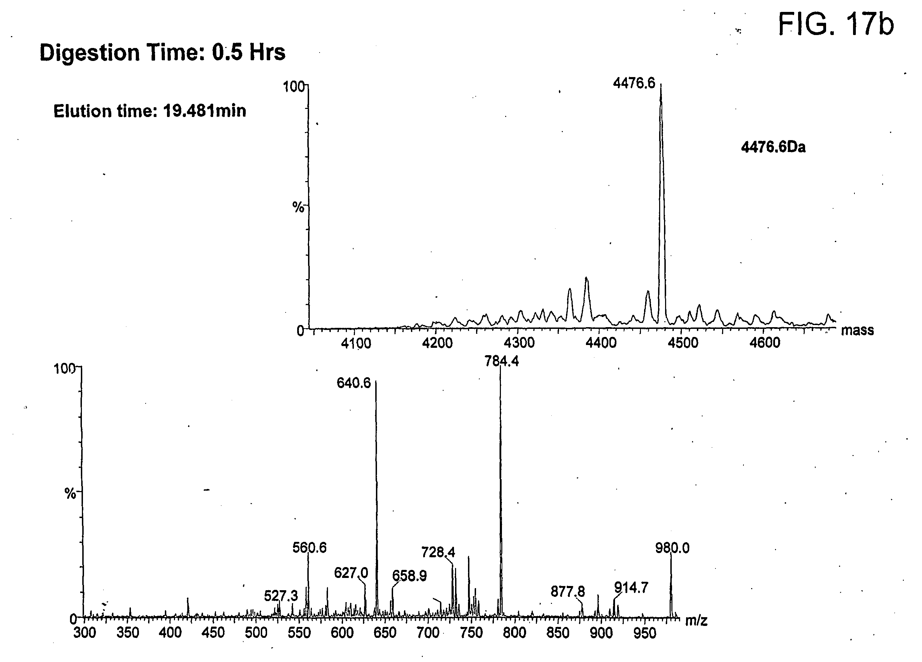

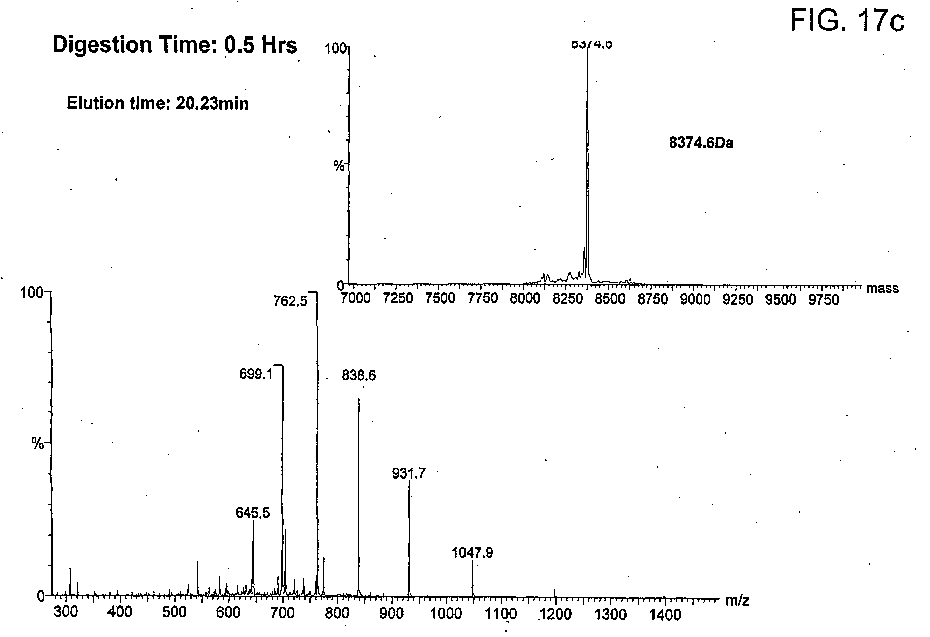

To evaluate the role of the linker used we carried out an *in vitro* digestion of the protein with cathepsin D at a ratio of ~400:1 (w/w) and followed the cleavage by mass spectrometry as a function of time (FIG. 16). The first cleavage occurred at the linker by around 30 minutes of treatment with the protease, giving two peptides of 3914.4Da (FIG. 17a) and 4477Da (FIG. 17b), which corresponded to M (1-34) L (expected mass 3915.5Da) and L (35-71) H (expected mass 4475Da) peptides respectively although the majority of the protein was still intact (FIG. 17c), which justified our design of the protein. Subsequently, the protein was cleaved at other sites as mapped by mass spectrometry till 2H of digestion (FIG's 18a-e, 19).

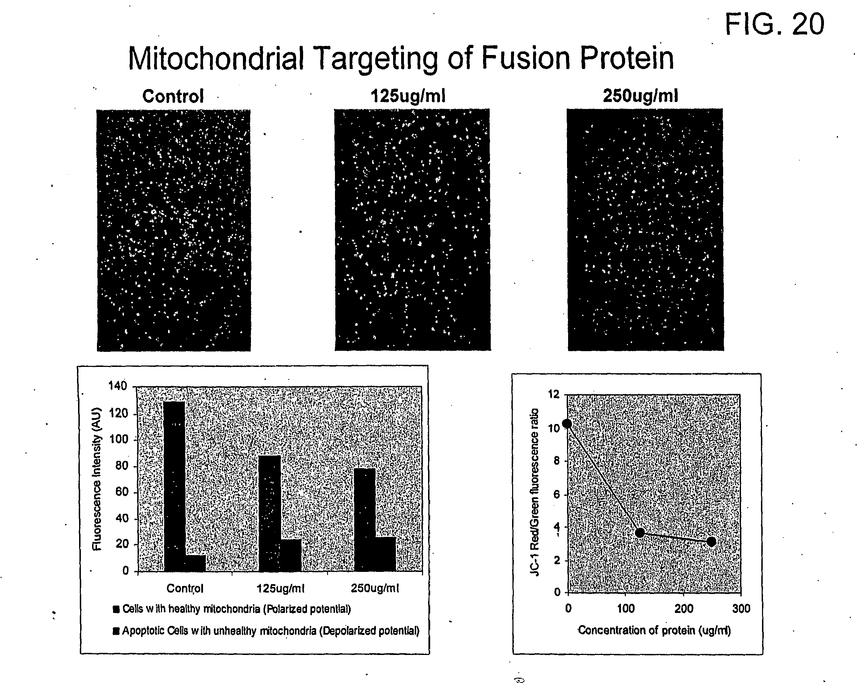

After having determined the role of the targeting VIP peptide and the cathepsin cleavable linker we investigated the role of the apoptotic BID peptide. In this regard, we monitored the mitochondrial membrane potential of the cells on treatment with fusion protein using JC-1 dye. It was found that the cytotoxic effect of the fusion protein was mediated by its ability to induce programmed cell death by decreasing the mitochondrial membrane potential in A549 cells exposed to fusion in a dose dependent manner with an increase in the population of cells with green fluorescence (FIG. 20a-d).

## DETAILED DESCRIPTION OF THE INVENTION

As mentioned above, the present invention relates to novel fusion proteins, pharmaceutical compositions comprising said fusion proteins and use of the protein as a therapeutic agent for treatment of cancer of various origins and as diagnostic marker for detection of tissue overexpressing the receptors of polypeptide A. For clarity of discussion, the specific compositions and uses described herein are exemplified using VIP and BH3 domains of Bcl2 family protein linked by a cancer specific protease cleavable linker; they are merely illustrative for the practice of the invention. Analogous procedures and techniques are equally applicable to constructing other fusion proteins between any cell-specific targeting moiety, protease cleavable linker and apoptosis-inducing moiety.

The fusion proteins of the present invention may be produced by fusion of a coding sequence of a cell-specific targeting moiety and a coding sequence of an apoptosis inducing domain with a linker as hereinbefore described, under the control of a regulatory sequence which directs the expression of the fusion polynucleotide in an appropriate host cell. The fusion of two full-length coding sequences can be achieved by methods well known in the art. It is preferred that a fusion polynucleotide contain only the AUG translation initiation codon at the 5' end of the first coding sequence without the initiation codon of the second coding sequence to avoid the production of two encoded products. In addition, a leader sequence may be placed at the 5' end of the polynucleotide or a peptide tag at the 3' end of the polynucleotide in order to target the expressed product to a specific site or compartment within a host cell to facilitate secretion or subsequent purification after gene expression. The tag may subsequently be cleaved or retained depending on the use.

**TABLE I: SEQUENCE IDENTITIES**

| Sequence ID | Sequence |
|---|---|
| 1 | 5'-CAT AGT GAT GCA GTG TTT ACC GAT AAT TAT ACG CGC CTT CGT AAG CAG ATG GCG GTT AAA AAG TAC TTG AAT AGC ATT CTG AAC- 3' |
| 2 | 5'- GTT CAG AAT GCT ATT CAA GTA CTT TTT AAC CGC CAT CTG CTT ACG AAG GCG CGT ATA ATT ATC GGT AAA CAC TGC ATC ACT ATG –3' |
| 3 | 5'-TCT CTG CTG ATC GCC AGA CGC ATG CCG AAC TTC AAC GAA GAT ATA ATC CGA AAT ATT GCG CGT CAC CTG GCT CAA GTC GGT GAT TCA ATG GAC CGT -3' |
| 4 | 5'- ACG GTC CAT TGA ATC ACC GAC TTG AGC CAG GTG ACG CGC AAT ATT TCG GAT TAT ATC TTC GTT GAA GTT CGG CAT GCG TCT GGC GAT CAG CAG AGA - 3' |
| 5 | 5'- CAT AGT GAT GCA GTG TTT ACC GAT AAT TAT ACG CGC CTT CGT AAG CAG ATG GCG GTT AAA AAG TAC TTG AAT AGC ATT CTG AAC GGA TCC TCT CTG CTG ATC GCC AGA CGC ATG CCG AAC TTC AAC GAA GAT ATA ATC CGA AAT ATT GCG CGT CAC CTG GCT CAA GTC GGT GAT TCA ATG GAC CGT CTC GAG CAC CAC CAC CAC CAC CAC -3' |
| 6 | AAT CAT ATG CAT AGT GAT GCA GTG TTT ACC GA |
| 7 | AAT GGA TCC GTT CAG AAT GCT ATT CAA GTA CTT |
| 8 | AAT GGA TCC TCT CTG CTG ATC GCC AGA CGC |
| 9 | AAT CTC GAG ACG GTC CAT TGA ATC ACC GAC TT |
| 10 | MHSDAVFTDNYTRLRKQMAVKKYLNSILNGSSLLIARRMPNFNEDIIRNIARHL AQVGDSMDRLEHHHHHH |

EP 1 957 524 B1

(continued)

| Sequence ID | Sequence |
|---|---|
| 11 | SLLIARRMPNFN |

The two coding sequences can be fused using a flexible protease site containing polylinker composed of S L L I A R R M P N F N (SEQ ID NO: 11 of Table I) which is designed to be recognized by cancer specific proteases and cleaved at a consensus site. Other linkers known in the art include ALALA [Studer M., et al., Bioconjug Chem., 1992, 3 (5), 424-9; Pan C. et al., Cancer Res, 2003, 63(17), 5526-5531; Trouet A. et al., PNAS, 1982, 79(2), 626-629; Moody T. W. et al., Life Sci, 2002,71(9), 1005-14]. The term "linker sequence" as used herein refers to an internal amino acid sequence within the protein encoded by the nucleic acid molecule of the invention which contains residues linking the targeting moiety and apoptotic domain so as to render the apoptotic domain incapable of exerting its toxic effect outside the target cell. Also the linker sequence should not interfere with the role of the targeting moiety in cell binding and internalization into lysosomes. When the linker sequence is cleaved the apoptotic domain becomes active or toxic.

Targeting a drug to a specific pharmacological site should enhance its therapeutic effectiveness and bioavailability thus reducing any toxic side effects, without any loss in drug efficiency. The fusion protein of the invention is composed of a cell-specific targeting moiety and an apoptosis-inducing moiety linked by a linker that has site for cancer specific proteases as hereinbefore described. The cell-specific targeting moiety confers cell-type specific binding to the molecule, and it is chosen on the basis of the particular cell population to be targeted. A wide variety of proteins are suitable for use as cell-specific targeting moieties like ligands for receptors to neuropeptides that are over-expressed in cancer cells as compared to normal cells, which are the subject of the present invention.

Since a large number of cell surface receptors have been identified in tumour cells of various lineages, ligands or antibodies specific for these receptors may be useful as cell-specific targeting moieties. In the present work, VIP was used as the cell-specific targeting moiety in a fusion protein to target VPAC 1 receptor expressing cells. Tumors expressing VPAC1 receptors include the most frequently occurring malignant epithelial neoplasms, such as cancers of the lung, stomach, colon, rectum, breast, prostate, pancreatic ducts, liver, and urinary bladder [Reubi J.C. et al., Cancer Res. 2000, 60, 3105-3112].

The BH3 domain of the pro-apoptotic proteins in the Bcl2 family specifically Bak, Bax, Bim, Bid and Bad is particularly suitable for use as the apoptosis-inducing moieties in the present invention. The BH3 domain of Bcl2 family protein is sufficient to induce apoptosis and has inherent advantage over the complete pro-apoptotic proteins. Bax BH3 peptide is more potent than full length Bax protein at inducing Cytochrome c from isolated mitochondria of Bcl-2-over-expressing cells, [Shangary S. et al., Biochemistry, 2002, 41 (30), 9485-95]. Such domains of human proteins are expected to have reduced immunogenicity over many immunotoxins composed of bacterial toxins. In a specific embodiment illustrated by working examples, the BH3 domain of BID coding sequence is fused with a VIP coding sequence through a specific flexible linker for the production of a fusion protein (SEQ ID: 10 of Table I). The nucleotide sequences encoding these proteins are known in the art, and thus cDNA clones can be readily obtained for fusion with a coding sequence for a cell-specific targeting moiety in an expression vector.

The recombinant fusion proteins of the invention have a targeting moiety of a neuropeptide selected from vasoactive intestinal peptide (VIP), somatostatin (SST), Gastrin releasing peptide (GST), Bombesin (BOM), or Substance P (Sub P) linked to an apoptotic-domain by a synthetic linker sequence having the amino acid sequence S L L I A R R M P N F N (SEQ ID No. 11), which may be cleaved specifically by a protease localized in cells or tissues affected by a specific disease to liberate the apoptotic domain which consists of a BH3 domain of a pro-apoptotic protein selected from Bak, Bax, Bim, Bid and Bad thereby destroying the diseased cells or tissues. The term diseased cell as used herein, includes cells affected by cancer. The cleavage recognition site of the invention is known to encode a cleavage recognition site for the cancer specific proteases. Other sequences encoding cleavage recognition sites could be identified by testing the expression product of the sequence for susceptibility to cleavage by the respective protease.

Degradable linkers explored for reducing toxicity to normal tissues from peptide conjugates include esters, thioethers, disulfides, amides, and hydrocarbon chains. Peptide linkers are preferred because of their stability in the circulation. Cathepsins are cysteine proteases found in all mammalian cell lysosomes. It is of interest in the potential utility of these linkers to fuse targeting and apoptotic moieties to target metastatic sites that over-express cathepsin (Elliott E. et al., Perspect. Drug Discov. Design, 1996, 6,12-32). The targeting moiety of a neuropeptide as hereinbefore described is linked to an apoptotic-domain as hereinbefore described by a synthetic linker sequence protease site containing polylinker composed of S L L I A R R M P N F N (SEQ ID NO: 11 of Table I) which is designed to be recognized by cancer specific proteases and cleaved at a consensus site.

Other known lysosomally cleavable peptides, specifically Gly-Phe-Leu-Gly [Kopecek J. et al., J. Control. Release, 1987, 6, 315-327] and Ala-Leu-Ala-Leu, [Trouet A. et al., Proc. Natl. Acad. Sci (U.S.A.), 1982, 79, 626-629] were judged to be less suitable as linkers for two reasons: (1) lysosomal drug release is relatively slow, and (2) the hydrophobicity of the linkers would likely cause precipitation or aggregation of protein during isolation and purification of fusion proteins.

The protein of the invention may be prepared using recombinant DNA methods. The nucleic acid molecule encoding the fusion protein of the invention may be cloned by amplifying the fragments thereof, constituting the fusion partners and ligating it into a cloning vector. Accordingly, the nucleic acid molecules encoding the fusion protein of the present invention may be incorporated in a known manner into an appropriate expression vector, which ensures good expression of the protein. The gene product itself may contain deletions, additions or substitutions of amino acid residues within a fusion sequence, which result in a silent change thus producing a functionally equivalent fusion protein. Specific initiation signals may also be required for efficient translation of the inserted fusion protein coding sequence. These signals include the ATG initiation codon and adjacent sequences.

A recombinant expression vector may be constructed to contain a nucleic acid molecule encoding the fusion protein of the invention, or a fragment thereof, and the necessary regulatory sequences for the transcription and translation of the inserted protein sequence. The recombinant vector may also contain a selectable marker gene, which facilitates the selection of host cells transformed or transfected with a recombinant molecule encoding the fusion protein of the invention. The recombinant expression vector may also contain a gene which encodes a fusion moiety which provides increased expression of the recombinant protein: increased solubility of the recombinant protein; and aid in the purification of the target recombinant protein by acting as a ligand in affinity purification. Suitable host cells include a wide variety of prokaryotic and eukaryotic host cells. Suitable bacterial expression vectors preferably comprise a promoter which functions in the host cell, one or more selectable phenotypic markers, and a bacterial origin of replication. Expression vectors include bacteriophages such as lambda derivatives or plasmids such as pBR322 (Bolivar et al., Gene, 1977, 2, 9S) or pUC series [Vieir et al., Gene, 1982, 19, 259-268]. Typical fusion expression vector or inducible non-fusion expression vectors including, but not limited to pTrc [Amann et al., Gene, 1988, 69, 301-315] and pET 22B (Studier et al., Gene Expression Technology: Methods in Enzymology, Academic Press, San Diego, Calif., 1990, 185, 60-89] may be used. In addition, a host cell strain may be chosen which modulates the expression of the inserted sequences, or modifies and processes the gene product in the specific fashion desired. Such modifications {e.g., glycosylation) and processing (e.g., cleavage) of protein products may be important for the function of the protein.

The recombinant protein of the invention can be isolated from suitable hosts using the methods described in the art. The fusion proteins can be tagged and purified by art-known techniques such as affinity chromatography, high performance liquid chromatography (HPLC), ion exchange chromatography, gel electrophoresis, and the like. For affinity chromatography purification, resin/any antibody, which specifically binds the protein, may be used.

Once a fusion protein is expressed and purified, its identity and functional activities can be readily determined by methods well known in the art. For example, the purity and the mass of the protein can be determined by Liquid chromatography-mass spectroscopy, antibodies to the two moieties of the protein may be used to identify the protein in Western blot analysis. In addition, the fusion protein can be tested for specific binding to target cells in binding assays using direct or indirect immunochemical methods.

The fusion protein of the invention targets specific cell types in a cell mixture, and eliminates the target cells by inducing apoptosis. The fusion protein of the invention is also useful as a diagnostic reagent. For example, fusion protein may be used to detect the tissue origin of a VPAC1 carcinoma. The binding of a fusion protein to a target cell can be readily detected by using a secondary antibody specific for the apoptosis-inducing moiety. In that connection, the secondary antibody or the fusion protein can be linked to a detectable label such as fluorescein, an enzyme or a radioisotope to facilitate the detection of binding of the fusion protein in a cell.

The fusion proteins of the invention can be used alone or with chemotherapeutic agents. After internalization and subsequent cleavage of fusion protein in the lysosome, fusion protein would release the BH3 domain of BID peptide. The freed BID peptide would then cause a reduction in the mitochondrial membrane potential, leading to the release of downstream death effectors that would cause apoptosis. However a second apoptotic signal, in the form of a chemotherapeutic agent at a lower dose could substantially elevate the levels of proapototic BID, which could kill the cells protected by Bcl-2 or Bcl$_{XL}$. Thus, reducing the side effects associated with the chemotherapeutic without compromising on its efficacy.

The fusion protein of the invention may be administered to a subject *per se* or in the form of pharmaceutical compositions for the treatment of cancer of various origins.

Pharmaceutical compositions were found to be useful for inhibiting the multiplication of cancer of various origins. In particular, the pharmaceutical compositions are found to be useful in treatment ovary, pancreas, head and neck, squamous cell, gastrointestine, breast, prostate, and non-small cell lung cancers.

The pharmaceutical compositions comprising the fusion protein may contain pharmaceutically acceptable carriers.

Therapeutically effective composition of fusion protein of the invention may be administered systematically to the mammal. Systemic administration refers to oral, rectal, nasal, transdermal, and parental (i.e., intramuscular, intravenous and subcutaneous). In accordance with good clinical practice, it is preferred to administer the composition at a dose that will produce anticancer effects without causing undue harmful side effects.

The composition may be administered either alone or as a mixture with other chemotherapeutic agents. Such chemotherapeutic agents could belong to various classes or categories of antineoplastic agents such as alkaloids like docetaxel, etoposide, irinotecan, paclitaxel, topotecan etc.; alkylating agents like busulfan, improsulfan etc.; anthracycline glycosides like daunarubicin, doxorubicin, epirubicin, idarubicin etc.; platinum complexes like carboplatin, cisplatin, oxaliplatin etc.; antimetabolites like methotrexate, edatrexate, purine analogs like fludrabine, cladribine, pyrimidine analogs like cytarabine, decitabine, fluorouracil, tegafur, gemcitabine etc.

The invention will be further described with respect to the following examples; the scope of the invention is not to be limited thereby.

**EXAMPLE 1**

**CONSTRUCTION OF VIP-BH3 BID CODING SEQUENCE**

A plasmid for the expression of VIP-BH3 BID fusion protein under the control of the T7 promoter was constructed as shown in FIG.3, which carried the fusion gene (SEQ ID: 5 of Table I). [Fishman et al., Biochem, 1994, 33, 6235-6243]. A cDNA encoding human VIP and BH3 BID/BAK was obtained by reverse transcription polymerase chain reaction (RT-PCR), using RNA isolated from HT-29 cells grown to logarithmic phase in presence of 10% Fetal Bovine Serum (FBS). Total RNA was isolated and was reverse transcribed into first strand cDNA, using the reverse transcription system (Promega, USA) under conditions recommended by the manufacturer. The cDNA was diluted to a total volume of 1 ml with 10 mM Tris-HCl pH 7.6, 1 mM EDTA and stored at 4° C.

An oligonucleotide encoding human BH3 BID/VIP was also synthesized chemically and purified by Poly acrylamide gel Electrophoresis (PAGE). The VIP encoding fragment was amplified using sense and antisense templates (SEQ ID NO: 1, 2 of Table I) with a pair of synthetic oligonucleotide primers: 5' AATCATATGCATAGTGATGCAGTGTTTACCGA 3' (SEQ ID NO: 6) (sense) and 5' AATGGATCCGTTCAGAATGCTATTCAAGTACTT 3' (SEQ ID NO: 7 of Table I) (antisense). The BH3 BID encoding fragment was amplified using sense and antisense templates (SEQ ID NO: 3, 4 of Table I) with a pair of synthetic oligonucleotide primers: 5' AATGGATCCTCTCTGCTGATCGCCAGACGC 3' (SEQ ID NO: 8 of Table I) (sense) and 5' AATCTCGAGACGGTCCATTGAATCACCGACTT 3' (SEQ ID NO: 9 of Table I) (antisense). The reaction mixture was incubated in a DNA thermal cycler for 25 cycles. Each cycle consisted of 30 sec. at 94° C., 30 sec. at 60° C. and 30 sec. at 72° C. The BH3 BID/VIP encoding fragment was digested with BamH1 enzymes and ligated. The fusion gene fragment was amplified by PCR using the flanking primers and cut with Nde 1 and Xho 1. The digested fusion fragment was ligated to pET22B expression vector. The resulting plasmid, designated p-fusion protein (SEQ ID NO: 5 of Table I), contained the human VIP coding sequence fused to the 5' end of the human BH3 BID coding sequence with the linker sequence in the middle. The plasmid was confirmed by restriction endonuclease digestion and DNA sequence analysis. The nucleotide and deduced amino acid sequences (SEQ ID NOS: 5, of Table I) of the fusion molecule referred to as fusion protein are disclosed in FIG.2.

**EXAMPLE 2**

**PROTEIN EXPRESSION AND PURIFICATION**

The p-fusion protein plasmid containing the fused coding sequences was transformed into E. coli strain BL21 (DE3) and the fusion protein (SEQ ID NO: 10) was expressed. A pellet of expressing cells was suspended in 10 mM Tris-HCl (pH 8.0) buffer containing Protease inhibitor cocktail (Sigma Chemical Co., St.Louis, MO, USA), sonicated (six 45-s bursts) and centrifuged at 13,000rpm for 10 min. The supernatant (soluble fraction) was removed and kept for analysis. The pellet was denatured in extraction buffer: 10 mM Tris-HCl-sodium chloride buffer (pH 8.0) containing 6M Guanidium chloride, and stirred for 30 min. at 4° C. The suspension was cleared by centrifugation at 13,000rpm for 10 min. and the pellet discarded. The supernatant was incubated with NiNTA resin (Qiagen, Hilden, Germany) and loaded on to a mini column. After washing the resin with 5 column volumes of wash buffer, the bound protein was eluted as per manufacturer's instruction. The protein profiles of various fractions (load, wash fractions, Elution fractions) were characterized by gel electrophoresis (FIG.8, 9).

**EXAMPLE 3**

**WESTERN BLOT ANALYSIS**

The electrophoresis samples were transferred onto nitro-cellulose and immunoblotted. The blot was probed with Anti-human VIP, anti-human BID, and anti-Hexahistidine tag at dilutions of 1: 1500, 1:3000, 1:3000, respectively. Anti-human VIP, anti-human BID was obtained from Santacruz (Santa Cruz Biotechnology, Inc, Santa Cruz, CA., USA.) and anti- Hexahistidine tag was obtained from Sigma (Sigma Chemical Co., St.Louis, MO, USA.).

## EXAMPLE 4

### LIQUID CHROMATOGRAPHY-MASS SPECTROMETERY (LC-MS) STUDIES

LC/MS analyses were carried out using a Waters 2695 high-perfomance liquid chromatography (HPLC) system (Milford, USA) and Micromass Quattro Micro mass spectrometer (Micromass, UK) using a ZORBAX Eclipse XDB-C18 (4.6 X150 mm, 5$\mu$M) column. Mobile phase solvents were 0.1% HCOOH in Milli Q Water (A) and 0.1% HCOOH in Acetonitrile (B). Fusion protein was eluted using a linear gradient program: 0 min 100% A; 60 min 100% B; 60-75 min 100% A, at a constant flow rate of 1.2mL min$^{-1}$, and a volume of 50$\mu$l was directed to the detector of the mass spectrometer. A valco flow splitter was introduced in to the HPLC solvent flow path between the UV-Visible detector and the mass spectrometer. The column temperature was maintained at 25°C.The injection volume was kept at 20$\mu$l. Detection of chromatographic eluants was monitered at 220nm. Positive ESI was utilized for the ionization of fusion protein in full-scan acquisition mode for intact molecular ion analyses and product ion mode was used for selective fragmentation of molecular ions. The capillary and cone voltages were set at 3.56 kV and 30 V, respectively. Nitrogen was used as desolvation gas at a flow rate of 500 L h$^{-1}$. The ESI source was maintained at 100°C and the desolvation temperature at 120°C to maintain the fragmentation of intact molecular ions. The data were acquired and processed using MassLynx 4.0 software (Micromass, UK).

## EXAMPLE 5

### CIRCULAR DICHROISM (CD) STUDIES

CD measurements were carried out on a Jasco J-715 spectropolarimeter at 25C. The protein concentration was kept at 10 $\mu$M. A path length of 10 mm was used, and the spectra were averaged over four scans at a scan speed of 10 nm/min. The spectra were recorded at varying dilutions of trifluoroethanol (TFE) and sodium dodecyl sulpahte (SDS). The spectra were corrected for baseline with respective controls.

## EXAMPLE 6

### CYTOTOXICITY ASSAY

Cells were seeded in 96-well microplates, followed by the addition of various concentrations of the fusion protein. After a 24-hour incubation, the MTT assay was performed according to the method of Mosmann [Mosmann T, J Immunol. Methods, 1983,55-63]. Serial dilutions of chemotherapeutic agents prepared by the Multiplication method were placed in a 96-well microplate. Since the cell lines employed in the present study had different proliferation rates, the number of cultured cells was adjusted to a density that allowed the untreated control cells to grow exponentially. That is, A549 was inoculated into the microplate wells at a density of 8,500 cells/well and the other cell lines were inoculated at a density ranging from 5,000 - 12,500 cells/well. After exposure of the cells to the test drug for desired time at 37C, 20ul of 3-(4,5-dimethylthiazol-2-yl)-2,5-diphenyltetrazolium bromide (MTT; Sigma Chemical Co., St.Louis, MO, USA) solution in PBS (5mg/ml) was added, and the cells were incubated for another 3h.Then 200$\mu$l of DMSO was added, and the absorbance was determined at a wavelength of 540nm using an ELISA reader. Results were expressed as the percent survival of the control experiments in which the cells were not exposed to any protein. All assays were carried out in triplicates.

## EXAMPLE 7

### RECEPTOR TARGETING

Cells were seeded in 96-well microplates, followed by the addition of various concentrations of the VIP antagonist VIP (10-28) Fragment for 30 minutes, followed by the addition of fusion protein at a fixed concentration. VIP (10-28) Fragment was allowed to remain in culture wells for whole of the experiment. After a 24-hour incubation, the MTT assay was performed as above.

## EXAMPLE 8

### IMMUNOCYTOCHEMICAL STAINING FOR CELL SURFACE RECEPTORS

Cultured (A549 human lung carcinoma) cells were detached with trypsin/EDTA and pelleted by low-speed centrifugation. The cells were resuspended in PBS. Cell suspensions were coated onto poly-L-lysine coated glass slides, dried and then fixed for 10 min in acetone at -20˚C. After drying for 2 h, slides were rinsed in PBS and then treated for 5 min with water/$H_2O_2$ in order to block endogenous peroxidase. Slides were again rinsed again in PBS and treated with normal rabbit and goat serum to block background staining. Slides were then incubated with antagonist, VIP $_{(10-28)}$ and/or fusion protein for 1 hr each. Slides were again washed with PBS and incubated with rabbit -anti-BID antibody (dilution 1 : 1000) or goat anti-VPAC 1 antibody (dilution 1 : 1000) for 1 h. Slides were again washed in PBS and incubated with rabbit anti-goat or goat anti-rabbit IgG peroxidase labelled antibodies for anti- BID and anti-VPAC 1 primary antibodies respectively, for 1 h followed by addition of the 3,3'-diaminobenzidine-tetrachloride (DAB) substrate and hydrogen peroxide in PBS. Slides were mounted in 70% glycerol and images were taken by Nikon Diaphot 300 (Nikon Co. Japan) inverted microscope.

## EXAMPLE 9

### CLEAVAGE AT THE LINKER SITE WITH RECOMBINANT HUMAN CATHEPSIN

One microgram of recombinant protein human cathepsin D (R&D Systems, Abingdon, UK), was activated by incubating it in 0.1 M NaOAc, 0.2 M NaCl, pH 3.5 for 30 minutes at 37˚ C. Thereafter, 250 ng activated cathepsin D were mixed with 100μg fusion protein in 0.1 M sodium acetate buffer, containing 0.2 M sodium chloride (pH 3.5) and incubated at 37˚C for required time and thereafter subjected to LC-MS, as described above.

## EXAMPLE 10

### MITOCHONDRIAL MEMBRANE POTENTIAL (MMP) ANALYSIS

Aliquots of cell suspensions were incubated with 4 μM of JC-1, a specific MMP dependent fluorescence dye [Hortelano S. et al.., FEBS Lett, 1997, 411: 77-82], for 30 min at 37˚C and washed once in PBS. Mitochondrial depolarization was qualitatively estimated by fluorescence microscopy with a long-pass filter. JC-1 exhibits dual fluorescence emission depending on MMP state. JC-1 forms aggregates in cells with a high red fluorescence (585 nm) indicating a normal MMP. MMP loss results in a reduction in red fluorescence with a concurrent gain in green fluorescence (530 nm) as the dye shifts from aggregates to monomeric state. Therefore, retention of the dye in the cells can be monitored through the increase in green fluorescence. The fluorescence was measures using Varioskan spectrofluorimeter (Thermo Electron Corporation, Waltham, MA.USA) and the data was converted to density plots.

## Claims

1. A fusion protein of formula,

$$A\text{-}B\text{-}C,$$

or

## C-B-A,

consisting of a fusion of three polypeptides A, B, and C, wherein polypeptide A is a neuropeptide selected from vasoactive intestinal peptide (VIP), somatostatin (SST), Gastrin releasing peptide (GST), Bombesin (BOM), or Substance P (Sub P), whose receptors are over expressed in tumor cells; polypeptide B is a linker having the amino acid sequence S L L I A R R M P N F N (SEQ ID No. 11); and polypeptide C consists of a BH3 domain of a pro-apoptotic protein selected from Bak, Bax, Bim, Bid and Bad.

2. The fusion protein as claimed in claim 1, wherein the receptors of polypeptide A are VPAC1, VPAC2, sst1, sst2, sst3, sst4, sst5, neuromedin B subtype (BB1), GRP subtype (BB2), BB3 subtype, BB4 subtypes, NK1, NK2, NK3 and LHRH.

3. A pharmaceutical composition comprising a therapeutically effective amount of fusion proteins of any preceding claim as therapeutic agents for use in the treatment of cancer of various origins.

4. The pharmaceutical composition as claimed in claim 3 further comprising pharmaceutically acceptable carriers.

5. The pharmaceutical composition as claimed in claim 3 wherein said cancer is selected from ovary, pancreas, head and neck, squamous cell, gastrointestine, breast, prostate, and non-small cell lung.

6. The fusion protein as claimed in any preceding claim wherein said fusion protein is useful as therapeutic agents for use in treatment of cancers of various origins either alone or in combination with other chemotherapeutic agents.

7. The fusion protein as claimed in any preceding claim wherein said fusion protein is produced by a chemical conjugation or recombinant DNA method.

8. The fusion protein as claimed in any preceding claim wherein said fusion protein is produced by recombinant DNA method.

9. The fusion protein as claimed in claim 1 wherein said fusion protein is useful as diagnostic marker for detection of tissue overexpressing the receptors of polypeptide A.


**Patentansprüche**

1. Fusionsprotein der Formel,

## A-B-C,

oder

## C-B-A,

bestehend aus einer Fusion von drei Polypeptiden A, B, und C, wobei Polypeptid A ein Neuropeptid ist, ausgewählt unter Vasoaktivem Intestinalem Peptid (VIP), Somatostatin (SST), Gastrin-freisetzendem Peptid (GST), Bombesin (BOM), oder Substanz P (Sub P), dessen Rezeptoren in Tumorzellen überexprimiert werden; Polypeptid B ein Linker mit der Aminosäuresequenz SLLIARRMPNFN (SEQ ID NO: 11) ist, und Polypeptid C aus einer BH3-Domäne eines unter Bak, Bax, Bim, Bid und Bad ausgewählten Pro-Apoproteins besteht.

2. Fusionsprotein nach Anspruch 1, wobei die Rezeptoren von Polypeptid A VPAC1, VPAC2, sst1, sst2, sst3, sst4, sst5, Neuromedin B Subtyp (BB1), GRP Subtyp (BB2), BB3 Subtyp, BB4 Subtypen, NK1, NK2, NK3 und LHRH sind.

3. Pharmazeutische Zusammensetzung, umfassend eine therapeutisch wirksame Menge von Fusionsproteinen nach einem der vorhergehenden Ansprüche als therapeutische Agenzien zur Verwendung bei der Behandlung von Krebs verschiedenen Ursprungs.

**4.** Pharmazeutische Zusammensetzung nach Anspruch 3, weiterhin umfassend pharmazeutisch akzeptable Täger.

**5.** Pharmazeutische Zusammensetzung nach Anspruch 3, wobei der Krebs ausgewählt ist unter Eierstockkrebs, Bauchspeicheldrüsenkrebs, Krebs des Kopfes und Nackens, Plattenepithelkarzinom, Gastrointestinalkrebs, Brustkrebs, Prostatakrebs, und nichtkleinzelligem Bronchialkarzinom.

**6.** Fusionsprotein nach einem der vorhergehenden Ansprüche, wobei das Fusionsprotein brauchbar ist als therapeutisches Agens für die Verwendung bei der Behandlung von Krebs verschiedenen Ursprungs, entweder alleine oder in Kombination mit weiteren chemotherapeutischen Agenzien.

**7.** Fusionsprotein nach einem der vorhergehenden Ansprüche, wobei das Fusionsprotein über eine chemische Konjugation oder ein Verfahren unter Verwendung rekombinanter DNA hergestellt wird.

**8.** Fusionsprotein nach einem der vorhergehenden Ansprüche, wobei das Fusionsprotein über ein Verfahren unter Verwendung rekombinanter DNA hergestellt wird.

**9.** Fusionsprotein nach Anspruch 1, wobei das Fusionsprotein brauchbar ist als diagnostischer Marker für den Nachweis von Gewebe, das Rezeptoren von Polypeptid A überexprimiert.

**Revendications**

**1.** Protéine de fusion de formule A-B-C ou C-B-A, consistant en la fusion de trois polypeptides A, B et C, dans laquelle le polypeptide A est un neuropeptide sélectionné parmi le peptide intestinal vasoactif (VIP), la somatostatine (SST), le peptide éliciteur de libération de gastrine (GST), la Bombésine (BOM) ou la Substance P (Sub P), dont les récepteurs sont surexprimés dans des cellules tumorales; le polypeptide B est un polypeptide de liaison présentant la séquence d'acides aminés suivante S L L I A R R M P N F N (SEQ ID No. 11); et le polypeptide C consiste en le domaine BH3 d'une protéine pro-apoptotique sélectionnée parmi les proétines Bak, Bax, Bim, Bid et Bad.

**2.** Protéine de fusion selon la revendication 1, **caractérisée en ce que** lesdits récepteurs du polypeptide A sont le récepteur VPAC1, le récepteur VPAC2, le récepteur sst1, le récepteur sst2, le récepteur sst3, le récepteur sst4, le récepteur sst5, le récepteur de la neuromédine de sous-type B (BB1), le récepteur à la GRP de sous type (BB2), le récepteur de sous-type BB3, le récepteur de sous-type BB4, le récepteur NK1, le récepteur NK2, le récepteur NK3 et le récepteur LHRH.

**3.** Composition pharmaceutique **caractérisée en ce qu'**elle comprend une quantité thérapeutiquement active d'une protéine de fusion selon l'une des revendications précédentes en tant qu'agent thérapeutique pour son utilisation dans le traitement de cancers d'origines diverses.

**4.** Composition pharmaceutique selon la revendication 3, **caractérisée en ce qu'**elle comprend de plus un agent de transport pharmaceutiquement acceptable.

**5.** Composition pharmaceutique selon la revendication 3, **caractérisée en ce que** ledit cancer est sélectionné parmi le cancer des ovaires, du pancréas, de la tête et du cou, des cellules squamous, gastrointestinal, du sein, de la prostate et du poumon hormis du poumon à petites cellules.

**6.** Protéine de fusion selon l'une des revendications 1 ou 2 pour son utilisation en tant qu'agent thérapeutique utile pour le traitement de cancers d'origines diverses seule ou en combinaison avec d'autres agents chimiothérapeutiques.

**7.** Protéine de fusion selon l'une des revendications 1, 2 ou 6, **caractérisée en ce que** ladite protéine de fusion est produite par combinaison chimique ou par la technique de l'ADN recombinant.

**8.** Protéine de fusion selon l'une des revendications 1, 2, 6 ou 7, **caractérisée en ce que** ladite protéine de fusion est produite par la technique de l'ADN recombinant.

9.  Protéine de fusion selon la revendication 1 pour son utilisation en tant que marqueur de diagnostic utile pour la détection de tissus sur-exprimant les récepteurs du polypeptide A.

# FIG. 1

## Strategy for Designing of Targeted VIP-BID Fusion Protein

A- Targeting Moiety Vasoactive Intestinal Peptide (VIP)

B- Flexible linker cleavable by cancer specific proteases

C- Apoptotic domain (BID peptide)

EP 1 957 524 B1

# FIG. 2

## Sequence of the Gene

5'- ATG CAT AGT GAT GCA GTG TTT ACC GAT AAT TAT ACG CGC CTT CGT AAG CAG ATG GCG GTT AAA AAG TAC TTG AAT AGC ATT CTG AAC GGA TCC TCT CTG CTG ATC GCC AGA CGC ATG CCG AAC TTC AAC GAA GAT ATA ATC CGA AAT ATT GCG CGT CAC CTG GCT CAA GTC GGT GAT TCA ATG GAC CGT CTC GAG CAC CAC CAC CAC CAC CAC -3'

## Sequence of the Protein

MHSDAVFTDNYTRLRKQMAVKKYLNSILN GSSLLIARRMPNFN EDIIRNIARHLAQVGDSMDRLE HHHHHH

A                    B                    C

EP 1 957 524 B1

FIG. 3

Cloning Strategy

# FIG. 4

## PCR Amplification of VIP and BID Fragments

Lane Details:
Lane 1 : PCR of VIP
Lane 2 : PCR of VIP
Lane 3 : PCR of Linker +BID
Lane 4 : PCR of Linker +BID
Lane 5: 100bp DNA marker

EP 1 957 524 B1

FIG. 5

# Ligation of VIP and Linker-BID Fragments

Lane Details:
Lane 1 : PCR of Linker +BID
Lane 2 : Ligated fragment
Lane 3 : Ligated Fragment
Lane 4 : 100bp DNA marker

EP 1 957 524 B1

FIG. 6

# Transformation of VIP-BID (Fusion Protein) Insert into Expression Vector

VBD-1
Fall out

Lane Details:
Lane 1&12: DNA ladder
Lane 2-10: Transformants

EP 1 957 524 B1

## FIG. 7

## Induction of different Clones of VIP-BID (Fusion Protein) using IPTG

Lane Details:
Lane 1 : Uninduced cell pellet Clone 1
Lane 2 : Induced cell pellet Clone 1
Lane 3 : Uninduced cell pellet Clone 2
Lane 4 : Induced cell pellet Clone 2
Lane 5 : Uninduced cell pellet Clone 3
Lane 6 : Induced cell pellet Clone 3
Lane 7 : Uninduced cell pellet Clone 4
Lane 8 : Induced cell pellet Clone 4
Lane 9 : Aprotinin standard

FIG. 8

## Elution Profile of Crude VIP-BID (Fusion Protein) Protein through Ni-NTA Column

**Elution profile of Crude VBD1 through Ni-NTA coulmn**

Fraction Details:
Fraction 1-6 : Flow through
Fraction 7-10 : Wash through
Fraction 11-18 : Eluted protein (VBD1)

EP 1 957 524 B1

FIG. 9

EP 1 957 524 B1

Elution Profile of Crude Fusion Protein through
Ni-NTA Column

Marker
Loaded protein
Flow through
Elution

FIG. 10

Immunoblots of Fusion Protein with Anti-VIP, Anti-Bid and Anti-His Antibodies

Anti-VIP    Anti-BID    Anti-His

Mass Spectra of Fusion Protein

FIG. 11

EP 1 957 524 B1

# FIG. 12

## Circular Dichroism Spectra of Fusion Protein

EP 1 957 524 B1

FIG. 13

Cytotoxicity of Fusion Protein in Different Cell Lines

| Cell Line | IC50 Value (uM) |
|-----------|-----------------|
| A549 | 5.1 |
| HT29 | 14.9 |
| NIH3T3 | >30 |

EP 1 957 524 B1

# Receptor Targeting of Fusion Protein

FIG. 14

**10uM VBD1**

**5uM VBD1**

(a)

(b)

EP 1 957 524 B1

# Receptor Targeting of Fusion Protein

FIG. 15

(a)

Control

(b)

(Anti-VPAC1)

(c)

Fusion protein-(Anti-BID)

(d)

VIP (10-28)-(Anti-VPAC1)

(e)

VIP (10-28)-Fusion protein-(Anti-BID)

(f)

Fusion protein-(Anti-VPAC1)

36

FIG. 16

Peptide Mapping of Fusion Protein Digested with Cathepsin D

# FIG. 17a

**Digestion Time: 0.5 Hrs**

**Elution time: 19.060min**

FIG. 17b

**Digestion Time: 0.5 Hrs**

**Elution time: 19.481min**

EP 1 957 524 B1

FIG. 17c

**Digestion Time: 0.5 Hrs**

**Elution time: 20.23min**

EP 1 957 524 B1

FIG. 18a

**Digestion Time: 2 Hrs**

**Elution time: 13.3min**

EP 1 957 524 B1

# FIG. 18b

**Digestion Time: 2 Hrs**

**Elution time: 12.66min**

**1663.66Da**

EP 1 957 524 B1

FIG. 18c

**Digestion Time: 2 Hrs**

Elution time: 12.894min

1136.8Da

1362.9Da

FIG. 18d

FIG. 18e

Peptide Mapping of Fusion Protein Digested with Cathepsin D

EP 1 957 524 B1

# Time Kinetics of Proteolytic Digestion of Fusion FIG. 19
## Protein using Cathepsin D

1          71

MHSDAVFTDNYTRLRKQMAVKKYLNSILNGSSLLIARRMPNFNEDIIRNIARHLAQVGDSMDRLEHHHHHH

8373Da

1        34      35         71

MHSDAVFTDNYTRLRKQMAVKKYLNSILNGSSLL     IARRMPNFNEDIIRNIARHLAQVGDSMDRLEHHHHHH

3915Da             4475Da

35      45     46        71

IARRMPNFNED

1362.5Da         3130.5Da

1     10     11          34

1136.2Da            2797Da

46       60   61      71

1663Da         1485.6Da

EP 1 957 524 B1

FIG. 20

# Mitochondrial Targeting of Fusion Protein

Control     125ug/ml     250ug/ml

■ Cells with healthy mitochondria (Polarized potential)

■ Apoptotic Cells with unhealthy mitochondria (Depolarized potential)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6645490 B

**Non-patent literature cited in the description**

- **Schmitt C.A.** *Nature Rev. Cancer,* 2003, vol. 3, 286
- **Reed J.C.** *Cancer Cell,* 2003, vol. 3, 17.2
- **Haldar S. et al.** *Proc Natl Acad Sci USA,* 1995, vol. 92, 4507
- **Strobel T. et al.** *Proc Natl Acad Sci USA,* 1996, vol. 93, 14094
- **Miao J. et al.** *Int. J. Oncol.,* 2004, vol. 25, 651
- **Gross A. et al.** *Genes Dev.,* 1999, vol. 13, 1899
- **Reed J.** *J. Clin. Oncol,* 1999, vol. 17, 2941
- **Abate-Shen C. et al.** *Genes Dev.,* 2001, vol. 4, 2410
- **Lowe S.W et al.** *Carcinogenesis,* 2000, vol. 21, 485
- **Johnson A.L.** *Endocrinology,* 1999, vol. 140, 5465
- **Cosulich S.C. et al.** *Curr. Biol,* 1997, vol. 7, 913
- **Lutz RJ.** *Biochem. Sci. Trans,* 2000, vol. 28
- **Minko T. et al.** *Clin. Outcomes,* 2001, vol. 3, 48
- **Dharap S.S. et al.** *Pharm Res,* 2003, vol. 20, 889
- **Schimmer, A.D. et al.** *Cell Death Differentiation,* 2001, vol. 8, 725
- **Zhao M. et al.** *Med. Res. Rev,* 2004, vol. 24, 1
- **Wender P.A et al.** *Proc. Natl. Acad. Sci USA,* 2000, vol. 97, 13003
- **Esposti M.D.** *Apoptosis,* 2002, vol. 7 (5), 433-40
- **Kamer I et al.** *Cell,* 2005, vol. 122 (4), 593-603
- **Walensky L. et al.** *Science,* 2004, vol. 305, 1466-70
- **Holinger et al.** *J. Biol. Chem.,* 1999, vol. 274, 13298-13304
- **Walsh M . et al.** *Blood,* 2002, vol. 99, 3439-3448
- **Walsh M. et al.** *Blood,* 2002, vol. 99, 3439-3448
- **Wang et al.** *Cancer Res,* 2000, vol. 60, 1498-1502
- **Ellerby et al.** *Nature Med,* 1999, vol. 1 (32), 1038
- **Mai et al.** *Cancer Res,* 2001, 7709-7712
- **Ichinose et al.** *Cancer Res,* 2002, vol. 62, 1433-1483
- **Thornburn et al.** *Clinical Cancer Res.,* 2003, vol. 9, 861-865
- **Brinkmann U. et al.** *Biochim. Biophys Acta.,* 1994, vol. 1198, 27-45
- **Aqeilan et al.** *FEBS letters,* 1999, 271-276
- **Reubi J.C et al.** *Cancer Res,* 2000, vol. 60, 3105-3112
- **Vaudry D. et al.** *Pharmacol Rev.,* 2000, vol. 52, 269-324
- **Robberecht P et al.** *J Clin Endocrinol Metab.,* 1993, vol. 77, 1235-1239
- **Vertongen P. et al.** *J Cell Physiol.,* 1996, vol. 167, 36-46
- **Oka H. et al.** *Am. J. Pathol.,* 1998, vol. 153, 1787-1796
- **Reubi J.C et al.** *Cancer Res.,* 2000, vol. 60, 3105-3112
- **Schally A.V.** *Cancer Res.,* 1988, vol. 48, 6977-6985
- **Reisine T. et al.** *Endocr Rev.,* 1995, vol. 16, 427-442
- **Hoyer D. et al.** *Trends Pharmacol Sci.,* 1995, vol. 16, 86-88
- **Cuttitta F. et al.** *Nature,* 1985, vol. 316, 823-825
- **Wada E et al.** *Neuron,* 1991, vol. 6, 421-430
- **Fathi Z. et al.** *J Biol Chem,* 1993, vol. 268, 5979-5984
- **Nagalla SR et al.** *Proc Natl Acad Sci USA,* 1995, vol. 92, 6205-6209
- **Hokfelt T et al.** *J Intern Med,* 2001, vol. 249, 27-40
- **Hennig I.M. et al.** *Int J Cancer,* 1995, vol. 61, 786-792
- **Friess H et al.** *Lab Invest,* 2003, vol. 83, 731-742
- **Irmer G. et al.** *Cancer Res,* 1995, vol. 55, 817-822
- **Emons G. et al.** *Hum Reprod,* 1994, vol. 9, 1364-1379
- **Halmos G. et al.** *J Urol,* vol. 163, 623-629

- **Straub B. et al.** *Clin Cancer Res,* 2001, vol. 7, 2340-2343
- **Grundker C et al.** *J Clin Endocrinol Metab,* 2002, vol. 87, 1427-1430
- **Vyas S.P. et al.** *Crit. Rev.Ther. Drug Carrier Syst,* 2001, vol. 18, 1-76
- **Langer M. et al.** *Curr. Med. Chem. Anti-Canc. Agents,* 2001, vol. 1, 71-93
- **Reubi J.C. et al.** *Cancer Res.,* 1996, vol. 56, 1922-1931
- **Reubi J.C.** *J Nucl. Med.,* 1995, vol. 36, 1846-1853
- **Carver J.A. et al.** *Eur. J. Biochem.,* 1990, vol. 187, 645-650
- **Letai A. et al.** *Cancer Cell,* 2002, vol. 2, 183
- **Walensky, L.D. et al.** *Science,* 2004, vol. 305, 1466
- **Gozes I. et al.** *Cell Mol. Neurobiol.,* 1995, vol. 15, 675
- **Studer M. et al.** *Bioconjug Chem.,* 1992, vol. 3 (5), 424-9
- **Pan C. et al.** *Cancer Res,* 2003, vol. 63 (17), 5526-5531
- **Trouet A. et al.** *PNAS,* 1982, vol. 79 (2), 626-629
- **Moody T. W. et al.** *Life Sci,* 2002, vol. 71 (9), 1005-14
- **Reubi J.C. et al.** *Cancer Res.,* 2000, vol. 60, 3105-3112
- **Shangary S. et al.** *Biochemistry,* 2002, vol. 41 (30), 9485-95
- **Elliott E. et al.** *Perspect. Drug Discov. Design,* 1996, vol. 6, 12-32
- **Kopecek J. et al.** *J. Control. Release,* 1987, vol. 6, 315-327
- **Trouet A. et al.** *Proc. Natl. Acad. Sci (U.S.A.),* 1982, vol. 79, 626-629
- **Bolivar et al.** *Gene,* 1977, vol. 2, 9S
- **Vieir et al.** *Gene,* 1982, vol. 19, 259-268
- **Amann et al.** *Gene,* 1988, vol. 69, 301-315
- **Studier et al.** Gene Expression Technology: Methods in Enzymology. Academic Press, 1990, vol. 185, 60-89
- **Fishman et al.** *Biochem,* 1994, vol. 33, 6235-6243
- **Mosmann T.** *J Immunol. Methods,* 1983, 55-63
- **Hortelano S. et al.** *FEBS Lett,* 1997, vol. 411, 77-82